# EUROPEAN PATENT APPLICATION

(11) **EP 1 334 971 A1**
(43) Date of publication of application: **13.08.2003**
(21) Application number: 01974795.5
(22) Date of filing: 11.10.2001
(51) Int. Cl.: C07D 401/12, A61K 31/4439, A61P 1/04, A61P 31/04, A61P 35/00

(54) **BENZIMIDAZOLE COMPOUNDS, PROCESS FOR PRODUCING THE SAME AND USE THEREOF**

(30) Priority: 12.10.2000 JP 2000316864
(71) Applicant: Takeda Chemical Industries, Ltd., Osaka-shi, Osaka 540-8645 (JP)
(72) Inventor: KAMIYAMA, Keiji, Osaka, 567-0037 (JP); SATO, Fumihiko, Suita-shi, Osaka 565-0872 (JP)
(74) Representative: Wright, Robert Gordon McRae
(86) International application number: JP0108943
(87) International publication number: WO02030920

(57) **Abstract**

A compound represented by the formula (I) 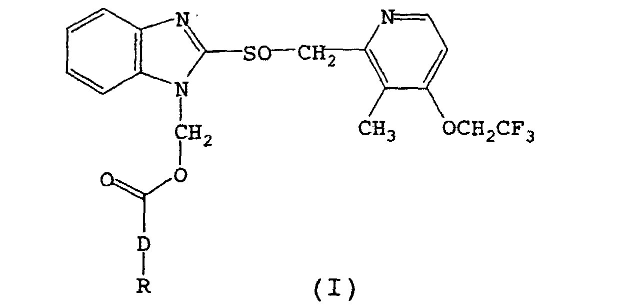 wherein each symbol is as defined in the specification, or a salt thereof (1) shows a superior anti-ulcer activity, a gastric acid secretion-suppressive action, a mucosa-protecting action, an anti-*Helicobacter pylori* action and the like in living organisms, (2) shows low toxicity, (3) is stable to acid (which obviates the need to formulate an enteric-coated preparation, thereby lowering the cost, and reduces the size of preparation to facilitate swallowing for patients having difficulty in swallowing), (4) shows faster absorption than enteric-coated preparations (rapid expression of gastric acid secretion-suppressive action), and (5) is sustainable.
According to the present invention, a benzimidazole compound, which has superior stability to acid and which is converted to a proton pump inhibitor in living organisms to show an anti-ulcer activity and the like, can be provided.

## Description

### Technical Field

The present invention relates to a benzimidazole compound, which is converted to a proton pump inhibitor in living organisms and shows an anti-ulcer activity and the like, a production method thereof and use thereof.

### Background Art

A proton pump inhibitor, 2-[[[3-methyl-4-(2,2,2-trifluoroethoxy)-2-pyridyl]methyl]sulfinyl]-1H-benzimidazole, and a salt thereof having an anti-ulcer activity are reported in JP-A-61-50978 and the like.

However, since the above-mentioned compounds are unstable to acids, for oral administration, they are formulated into an enteric-coated preparation, filled in a capsule and administered to prevent decomposition by gastric acid.

Therefore, the development of a prodrug of the above-mentioned compound, which is stable to acid and resists decomposition by gastric acid, has been desired, and such prodrug has been reported in US Patent No. 6,093,734. In addition, prodrugs of proton pump inhibitors other than the above-mentioned prodrugs have been disclosed in US Patent Nos. 4,045,563, 4,686,230, 4,873,337, 4,965,269, 5,021,433 and the like.

In view of the above situation, the development of a prodrug of a proton pump inhibitor having superior stability to acid has been desired.

It is therefore an object of the present invention to provide a benzimidazole compound having superior stability to acid, which is converted to 2-[[[3-methyl-4-(2,2,2-trifluoroethoxy)-2-pyridyl]methyl]sulfinyl]-1H-benzimidazole in living organisms and shows an anti-ulcer activity and the like, a production method thereof and use thereof.

### Disclosure of the Invention

The present inventors have first synthesized a compound represented by the following formula (I) and first found that this compound has unexpectedly superior stability to acid, gradually eliminates the substituent on the nitrogen atom of benzimidazole ring and affords a sustained acid secretion-suppressive action. Further studies based on these findings have resulted in the completion of the present invention.

According to the present invention, 2-[[[3-methyl-4-(2,2,2-trifluoroethoxy)-2-pyridyl]methyl]sulfinyl]-1H-benzimidazole is modified to give a prodrug (the compound of the formula (I)) stable to acid, which enables oral administration of the compound as a conventional tablet and the like without formulating an enteric-coated preparation. This has a consequence that the cost for formulating an enteric-coated preparation can be eliminated and the preparation of tablet and the like can be made smaller. A smaller preparation is advantageous in that it is easily swallowed by patients having difficulty in swallowing, particularly the elderly and children. In addition, absorption is rapid due to the absence of a sustained release effect afforded by enteric-coated preparations, expression of a gastric acid secretion-suppressive action is rapid, and alleviation of symptoms such as pain and the like is rapid. Furthermore, because the compound is gradually converted to a proton pump inhibitor in living organisms, a sustainable anti-ulcer agent and the like can be provided.

Accordingly, the present invention provides the following.
1) A benzimidazole compound represented by the formula (I) 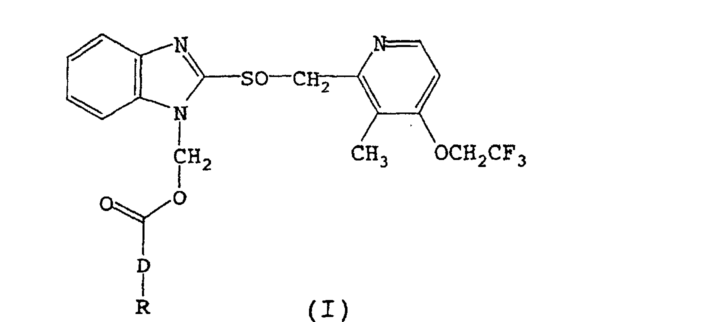 wherein D is an oxygen atom or a bond, and R is a hydrocarbon group optionally having substituents (hereinafter sometimes abbreviated as compound (I)), or a salt thereof.
2) The compound of the above-mentioned 1), wherein R is a C₁₋₆ alkyl group, a C₂₋₆ alkenyl group or a C₂₋₆ alkynyl group, which optionally has substituents selected from the group consisting of (i) a C₆₋₁₄ aryl group, (ii) a hydroxyl group, (iii) a halogen, (iv) a C₁₋₆ alkoxy group optionally substituted by a halogen, (v) a C₇₋₁₂ aralkyloxy group, (vi) a C₁₋₅ alkoxy-carbonyl group and (vii) an acylamino group, or a C₃₋₈ cycloalkyl group or a C₆₋₁₄ aryl group, which optionally has substituents selected from the group consisting of (i) a C₆₋₁₄ aryl group, (ii) a hydroxyl group, (iii) a halogen, (iv) a C₁₋₆ alkoxy group optionally substituted by a halogen, (v) a C₇₋₁₂ aralkyloxy group, (vi) a C₁₋₅ alkoxy-carbonyl group and (vii) a C₁₋₆ alkyl group optionally substituted by a halogen, or a salt thereof.
3) The compound of the above-mentioned 1), wherein R is a C₁₋₆ alkyl group optionally having substituents selected from the group consisting of (i) a C₆₋₁₄ aryl group, (ii) a hydroxyl group, (iii) a halogen and (iv) a C₁₋₆ alkoxy group optionally substituted by a halogen, (v) a C₇₋₁₂ aralkyloxy group, (vi) a C₁₋₅ alkoxy-carbonyl group and (vii) an acylamino group, or a C₃₋₈ cycloalkyl group or a C₆₋₁₄ aryl group, which optionally has substituents selected from the group consisting of (i) a C₆₋₁₄ aryl group, (ii) a hydroxyl group, (iii) a halogen, (iv) a C₁₋₆ alkoxy group optionally substituted by a halogen, (v) a C₇₋₁₂ aralkyloxy group, (vi) a C₁₋₅ alkoxy-carbonyl group and (vii) a C₁₋₆ alkyl group optionally substituted by a halogen, or a salt thereof.
4) The compound of the above-mentioned 1), wherein D is a bond and R is an alkyl group optionally having substituents or an aryl group optionally having substituents, or a salt thereof.
5) The compound of the above-mentioned 4), wherein R is (1) a C₁₋₆ alkyl group optionally having 1 to 5 substituents selected from the group consisting of (i) a C₆₋₁₄ aryl group, (ii) a hydroxyl group, (iii) a halogen, (iv) a C₁₋₆ alkoxy group optionally substituted by 1 to 5 halogens, (v) a C₇₋₁₂ aralkyloxy group and (vi) a C₁₋₅ alkoxy-carbonyl group, or (2) a C₆₋₁₄ aryl group optionally having 1 to 5 substituents selected from the group consisting of (i) a halogen, (ii) a C₁₋₆ alkyl group optionally substituted by 1 to 5 halogens, (iii) a C₆₋₁₄ aryl group, (iv) a hydroxyl group, (v) a C₁₋₆ alkoxy group optionally substituted by 1 to 5 halogens, (vi) a C₇₋₁₂ aralkyloxy group and (vii) a C₁₋₅ alkoxy-carbonyl group, or a salt thereof.
6) The compound of the above-mentioned 4), wherein R is (1) a C₁₋₆ alkyl group optionally substituted by a C₆₋₁₄ aryl group or (2) a C₆₋₁₄ aryl group, or a salt thereof.
7) The compound of the above-mentioned 4), wherein R is a phenyl group, or a salt thereof.
8) The compound of the above-mentioned 4), wherein R is a methyl group or a tert-butyl group, or a salt thereof.
9) The compound of the above-mentioned 1), which is an (R)-form represented by the formula 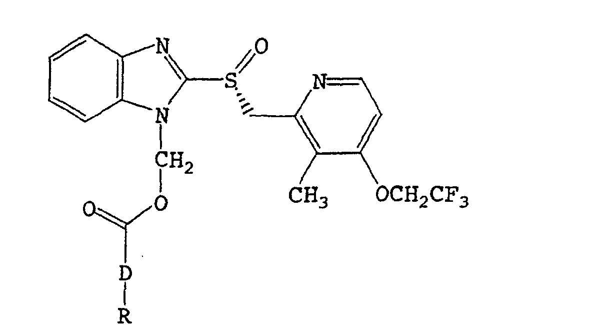 wherein each symbol is as defined in the above-mentioned 1, or a salt thereof.
10) A production method for the compound of the above-mentioned
   1) or a salt thereof, which comprises (1) condensing a compound represented by the formula (II) 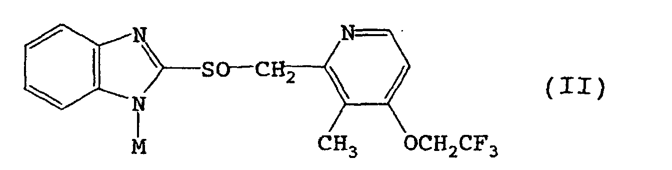 wherein M is a hydrogen atom, a metal cation or a quaternary ammonium ion (hereinafter sometimes abbreviated as compound (II)), or a salt thereof, with a compound represented by the formula (III): R-D-C(=O)-O-CH₂-X wherein X is a halogen, D is an oxygen atom or a bond, and R is a hydrocarbon group optionally having substituents (hereinafter sometimes abbreviated as compound (III)), or
   (2) condensing a compound represented by the formula (IV) 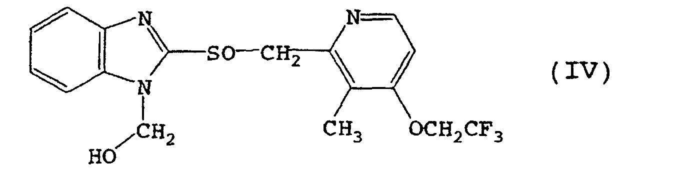 (hereinafter sometimes abbreviated as compound (IV)), with carboxylic acid represented by the formula: R-D-COOH wherein each symbol is as defined above or a reactive derivative thereof, or
   (3) subjecting a compound represented by the formula (V) 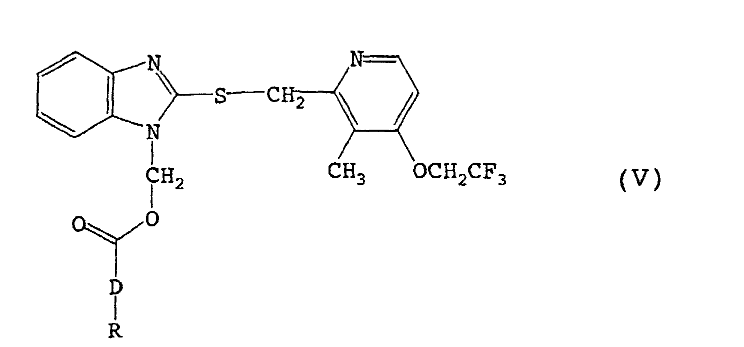 wherein each symbol is as defined above (hereinafter sometimes abbreviated as compound (V)), or a salt thereof, to oxidation reaction.
11) A pharmaceutical composition comprising the compound of the above-mentioned 1) or 4).
12) The pharmaceutical composition of the above-mentioned 11), which is an agent for the prophylaxis or treatment of digestive ulcer, gastritis, reflux esophagitis, NUD, gastric cancer, gastric MALT lymphoma, gastric hyperacidity or upper gastrointestinal hemorrhage.
13) A commercial package comprising a pharmaceutical composition of the above-mentioned 12) and written matter associated therewith, the written matter stating that the pharmaceutical composition can or should be used for the prophylaxis or treatment of digestive ulcer, gastritis, reflux esophagitis, NUD, gastric cancer, gastric MALT lymphoma, gastric hyperacidity or upper gastrointestinal hemorrhage.
14) The pharmaceutical composition of the above-mentioned 11), which is an agent for the eradication of *Helicobacter pylori*.
15) A commercial package comprising a pharmaceutical composition of the above-mentioned 14) and written matter associated therewith, the written matter stating that the pharmaceutical composition can or should be used for eradicating *Helicobacter pylori.*
16) An agent for the prophylaxis or treatment of digestive ulcer, gastritis, reflux esophagitis, NUD, gastric cancer, gastric MALT lymphoma, gastric hyperacidity or upper gastrointestinal hemorrhage, which comprises the compound of the above-mentioned 1) as an active ingredient.
17) An agent for the eradication of *Helicobacter pylori,* which comprises the compound of the above-mentioned 1) as an active ingredient.
18) A method for the prophylaxis or treatment of digestive ulcer, gastritis, reflux esophagitis, NUD, gastric cancer, gastric MALT lymphoma, gastric hyperacidity or upper gastrointestinal hemorrhage, which comprises administering the compound of the above-mentioned 1).
19) A method for eradicating *Helicobacter pylori,* which comprises administering the compound of the above-mentioned 1).
20) Use of the compound of the above-mentioned 1) for the production of an agent for the prophylaxis or therapy of digestive ulcer, gastritis, reflux esophagitis, NUD, gastric cancer, gastric MALT lymphoma, gastric hyperacidity or upper gastrointestinal hemorrhage.
21) Use of the compound of the above-mentioned 1) for the production of an agent for the eradication of *Helicobacter pylori*.

### Embodiment of the Invention

In the present invention, the "aryl group" is a monocyclic or condensed polycyclic aromatic hydrocarbon group, preferably an aromatic hydrocarbon group having 6 to 14 carbon atoms ("C₆₋₁₄ aryl group"). Examples thereof include phenyl, naphthyl, anthryl, phenanthryl and acenaphthylenyl, and preferred is an aromatic hydrocarbon group having 6 to 10 carbon atoms, and for R, phenyl is particularly preferable.

In the present invention, the "halogen" is fluorine, chlorine, bromine or iodine. The halogen as a substituent of the hydrocarbon group represented by R in the formula (I) is preferably fluorine or chlorine.

In the present invention, the "C₁₋₆ alkoxy group optionally substituted by halogen" is a linear or branched chain alkoxy group having 1 to 6 carbon atoms, which is optionally substituted by halogen (as defined above); preferably 1 to 5, more preferably 1 to 3, halogens. Examples of the C₁₋₆ alkoxy group include methoxy, ethoxy, n-propoxy, isopropoxy, n-butoxy, isobutoxy, sec-butoxy, tert-butoxy and the like, with preference given to an alkoxy group having 1 to 4 carbon atoms. As the C₁₋₆ alkoxy group optionally substituted by halogen, methoxy, ethoxy, methoxy, ethoxy, n-propoxy, isopropoxy, trifluoromethoxy and 2,2,2-trifluoroethoxy are preferable.

In the present invention, the "alkyl group" means a linear or branched chain alkyl group, preferably an alkyl group having 1 to 6 carbon atoms ("C₁₋₆ alkyl group"). Examples thereof include methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 3,3-dimethylpropyl, 2-ethylbutyl and the like, more preferably alkyl having 1 to 4 carbon atoms. For R, methyl, ethyl, isopropyl and tert-butyl are preferable, and tert-butyl is particularly preferable.

In the present invention, the "C₇₋₁₂ aralkyloxy group" is an aralkyloxy group having 7 to 12 carbon atoms wherein aryl group is as defined for the above-mentioned aryl group (preferably phenyl group) and the alkyl moiety is as defined for the above-mentioned "C₁₋₆ alkyl group". Examples thereof include benzyloxy, 1-naphthylmethyloxy, 2-naphthylmethyloxy and the like. Preferred is an aralkyloxy group having 7 to 11 carbon atoms, more preferably phenyl-C₁₋₄ alkyloxy group and particularly preferably benzyloxy.

In the present invention, the "C₁₋₅ alkoxy-carbonyl group" is an alkoxycarbonyl group wherein the alkoxy moiety is a linear or branched chain alkoxy group having 1 to 5 carbon atoms, which is exemplified by methoxycarbonyl, ethoxycarbonyl, propoxycarbonyl, isopropoxycarbonyl and the like. Preferred is an alkoxycarbonyl group wherein the alkoxy moiety has 1 to 4 carbon atoms, and particularly preferred are methoxycarbonyl and ethoxycarbonyl.

In the present invention, the "C₁₋₆ alkyl group optionally substituted by halogen" is a C₁₋₆ alkyl group (as defined above) optionally substituted by halogen(s) (preferably 1 to 5, more preferably 1 to 3), which is preferably methyl, ethyl, propyl, isopropyl or trifluoromethyl.

In the present invention, the "C₂₋₆ alkenyl group" is a linear or branched chain alkenyl group having 2 to 6 carbon atoms, which is exemplified by vinyl, n-propenyl, isopropenyl, n-butenyl, isobutenyl, sec-butenyl, tert-butenyl, n-pentenyl, isopentenyl, neopentenyl, 1-methylpropenyl, n-hexenyl, isohexenyl, 1,1-dimethylbutenyl, 2,2-dimethylbutenyl, 3,3-dimethylbutenyl, 3,3-dimethylpropenyl, 2-ethylbutenyl and the like. Preferred is an alkenyl group having 2 to 4 carbon atoms, and particularly preferred are vinyl, n-propenyl and isopropenyl.

In the present invention, the "C₂₋₆ alkynyl group" is a linear or branched chain alkynyl group having 2 to 6 carbon atoms, which is exemplified by ethynyl, n-propynyl(1-propynyl), isopropynyl(2-propynyl), n-butynyl, isobutynyl, sec-butynyl, tert-butynyl, n-pentynyl, isopentynyl, neopentynyl, 1-methylpropynyl, n-hexynyl, isohexynyl, 1,1-dimethylbutynyl, 2,2-dimethylbutynyl, 3,3-dimethylbutynyl, 3,3-dimethylpropynyl, 2-ethylbutynyl and the like. Preferred is an alkynyl group having 2 or 3 carbon atoms, and particularly preferred are ethynyl, 1-propynyl and 2-propynyl.

In the present invention, the "C₃₋₈ cycloalkyl group" is a linear or branched chain cycloalkyl group having 3 to 8 carbon atoms, which is exemplified by cyclopropyl, cyclobutyl, cyclopentyl, cyclohexyl, cycloheptyl, cyclooctyl and the like. Preferred is a cycloalkyl group having 5 to 7 carbon atoms, more preferred are cyclopentyl, cyclohexyl and cycloheptyl, and particularly preferred is cyclohexyl.

In the present invention, the "hydrocarbon group" encompasses an aliphatic or aromatic hydrocarbon group, wherein the aliphatic hydrocarbon group means a saturated or unsaturated linear, branched chain or cyclic hydrocarbon group. As the hydrocarbon group, a hydrocarbon group having 1 to 14 carbon atoms is preferable, which is exemplified by C₁₋₆ alkyl group, C₂₋₆ alkenyl group, C₂₋₆ alkynyl group, C₃₋₈ cycloalkyl group and C₆₋₁₄ aryl group. Preferred are C₁₋₆ alkyl group, C₃₋₈ cycloalkyl group and C₆₋₁₄ aryl group and more preferred are C₁₋₆ alkyl group and C₃₋₈ cycloalkyl group.

In the present invention, for example, the "acylamino group" is preferably alkanoylamino wherein the alkanoyl moiety preferably has 1 to 6 carbon atoms, such as acetylamino, formylamino, propionylamino, butylamino, isobutylamino, pentanoylamino, hexanoylamino and the like, alkenoylamino wherein the alkenoyl moiety preferably has 3 to 6 carbon atoms, such as propenoylamino, 1-butenoylamino, 2-butenoylamino, 1-pentenoylamino, 1-hexenoylamino and the like, cycloalkanoylamino preferably having 4 to 8 carbon atoms, such as cyclopropanoylamino, cyclobutanoylamino, cyclopentanoylamino, cyclohexanoylamino, cycloheptanoylamino and the like, and the like. The acylamino group is preferably alkanoylamino, particularly preferably acetylamino.

In the present invention, the "metal cation" is exemplified by alkali metal ion (e.g., Na⁺, K⁺, Li⁺, Cs⁺ and the like), with preference given to Na⁺ and Cs⁺.

In the present invention, the "quaternary ammonium ion" is exemplified by tetramethylammonium, tetraethylammonium, tetrapropylammonium, tetrabutylammonium and the like, with preference given to tetrabutylammonium.

The above-mentioned "hydrocarbon group" may be substituted, wherein the substituent is exemplified by C₆₋₁₄ aryl group, hydroxyl group, halogen, C₁₋₆ alkoxy group optionally substituted by halogen, C₇₋₁₂ aralkyloxy group, C₁₋₅ alkoxy-carbonyl group, C₁₋₆ alkyl group optionally substituted by halogen, amino group optionally substituted by C₁₋₆ alkyl group and the like.

The substituent for the "alkyl group optionally having substituents" is exemplified by aryl group, hydroxyl group, halogen, alkoxy group optionally substituted by 1 to 5 halogens, C₇₋₁₂ aralkyloxy group, C₁₋₅ alkoxy-carbonyl group and the like. The number of the substituents is 1 to 5, preferably 1 to 3.

The substituent for the "aryl group optionally having substituents" is exemplified by halogen, alkyl group optionally substituted by 1 to 5 halogens, aryl group, hydroxyl group, alkoxy group optionally substituted by 1 to 5 halogens, C₇₋₁₂ aralkyloxy group, C₁₋₅ alkoxy-carbonyl group and the like. The number of the substituents is 1 to 5, preferably 1 to 3.

The above-mentioned "C₁₋₆ alkyl group", "C₂₋₆ alkenyl group" and "C₂₋₆ alkynyl group" may be substituted, and the substituent is exemplified by (i) a C₆₋₁₄ aryl group, (ii) a hydroxyl group, (iii) a halogen, (iv) a C₁₋₆ alkoxy group optionally substituted by halogen, (v) a C₇₋₁₂ aralkyloxy group, (vi) a C₁₋₅ alkoxy-carbonyl group, (vii) an acylamino group, (viii) an amino group optionally substituted by C₁₋₆ alkyl group and the like. Of these, (i) to (vii) are preferable. The number of the substituents is 1 to 5, preferably 1 to 3.

The above-mentioned "C₃₋₈ cycloalkyl group" and "C₆₋₁₄ aryl group" may be substituted, wherein the substituent is exemplified by (i) a C₆₋₁₄ aryl group, (ii) a hydroxyl group, (iii) a halogen, (iv) a C₁₋₆ alkoxy group optionally substituted by halogen, (v) a C₇₋₁₂ aralkyloxy group, (vi) a C₁₋₅ alkoxy-carbonyl group, (vii) a C₁₋₆ alkyl group optionally substituted by halogen, (viii) an amino group optionally substituted by C₁₋₆ alkyl group and the like. Of these, (i)-(vii) are preferable. The number of the substituents is 1 to 5, preferably 1 to 3.

In the present invention, R is preferably a C₁₋₆ alkyl group, a C₂₋₆ alkenyl group or a C₂₋₆ alkynyl group, which optionally has substituents selected from the group consisting of (i) a C₆₋₁₄ aryl group, (ii) a hydroxyl group, (iii) a halogen, (iv) a C₁₋₆ alkoxy group optionally substituted by halogen, (v) a C₇₋₁₂ aralkyloxy group, (vi) a C₁₋₅ alkoxy-carbonyl group and (vii) an acylamino group, or a C₃₋₈ cycloalkyl group or a C₆₋₁₄ aryl group, which optionally has substituents selected from the group consisting of (i) a C₆₋₁₄ aryl group, (ii) a hydroxyl group, (iii) a halogen, (iv) a C₁₋₆ alkoxy group optionally substituted by halogen, (v) a C₇₋₁₂ aralkyloxy group, (vi) a C₁₋₅ alkoxy-carbonyl group and (vii) a C₁₋₆ alkyl group optionally substituted by halogen, more preferably (1) a C₁₋₆ alkyl group optionally having 1 to 5 substituents selected from the group consisting of (i) a C₆₋₁₄ aryl group, (ii) a hydroxyl group, (iii) a halogen, (iv) a C₁₋₆ alkoxy group optionally substituted by 1 to 5 halogens, (v) a C₇₋₁₂ aralkyloxy group and (vi) a C₁₋₅ alkoxy-carbonyl group, or (2) a C₆₋₁₄ aryl group optionally having 1 to 5 substituents selected from the group consisting of (i) a halogen, (ii) a C₁₋₆ alkyl group optionally substituted by 1 to 5 halogens, (iii) a C₆₋₁₄ aryl group, (iv) a hydroxyl group, (v) a C₁₋₆ alkoxy group optionally substituted by 1 to 5 halogens, (vi) a C₇₋₁₂ aralkyloxy group and (vii) a C₁₋₅ alkoxy-carbonyl group, still more preferably a C₁₋₆ alkyl group optionally having substituents selected from the group consisting of (i) a C₆₋₁₄ aryl group, (ii) a hydroxyl group, (iii) a halogen, (iv) a C₁₋₆ alkoxy group optionally substituted by halogen, (v) a C₇₋₁₂ aralkyloxy group, (vi) a C₁₋₅ alkoxy-carbonyl group and (vii) an acylamino group, or
a C₃₋₈ cycloalkyl group or a C₆₋₁₄ aryl group, which optionally has substituents selected from the group consisting of (i) a C₆₋₁₄ aryl group, (ii) a hydroxyl group, (iii) a halogen, (iv) a C₁₋₆ alkoxy group optionally substituted by halogen, (v) a C₇₋₁₂ aralkyloxy group, (vi) a C₁₋₅ alkoxy-carbonyl group and (vii) a C₁₋₆ alkyl group optionally substituted by halogen, and among them, R is preferably a C₁₋₆ alkyl group optionally substituted by C₆₋₁₄ aryl group or a C₆₋₁₄ aryl group, particularly a phenyl group, or methyl or tert-butyl group.

In the compound (I), a pharmacologically acceptable basic salt can be formed between an acidic group in a molecule and an inorganic base or an organic base etc, and a pharmacologically acceptable acid addition salt can be formed between a basic group in a molecule and an inorganic acid or an organic acid etc.

One of the preferable embodiments of compound (I) of the present invention is a compound wherein D is a bond and R is an alkyl group optionally having substituents or an aryl group optionally having substituents.

Examples of the inorganic basic salt of compound (I) include salt with alkali metal (e.g., sodium, potassium and the like), alkaline earth metal (e.g., calcium and the like), ammonia and the like, and the like, and examples of the organic basic salt of compound (I) include salt with dimethylamine, triethylamine, piperazine, pyrrolidine, piperidine, 2-phenylethylamine, benzylamine, ethanolamine, diethanolamine, pyridine, collidine and the like, and the like.

Examples of the acid addition salt of compound (I) include inorganic acid salt (e.g., hydrochloride, sulfate, hydrobromide, phosphate and the like), organic acid salt (e.g., acetate, trifluoroacetate, succinate, maleate, fumarate, propionate, citrate, tartrate, lactate, oxalate, methanesulfonate, p-toluenesulfonate and the like) and the like.

The compound (I) of the present invention encompasses hydrates. Examples of the "hydrate" include 0.5 hydrate - 5.0 hydrate. Of these, 0.5 hydrate, 1.0 hydrate, 1.5 hydrate and 2.0 hydrate are preferable.

The compound (I) of the present invention encompasses racemates and optically active compounds. As the optically active compound, such compound wherein one enantiomer is in enantiomer excess (e.e.) of not less than 90% is preferable, more preferably in enantiomer excess (e.e.) of not less than 99%. As an optically active form, an (R)-form represented by the formula 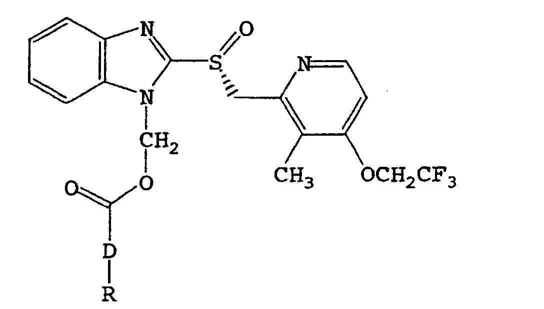 wherein each symbol is as defined above, is preferable.

The compound (I) can be produced by a method known per se, such as the methods described in US Patent Nos. 4,873,337 and 5,021,433, or a similar method, such as the following methods A to C.

### Method A

The compound (II) or a salt thereof is condensed with compound (III), whereby compound (I) or a salt thereof can be obtained. 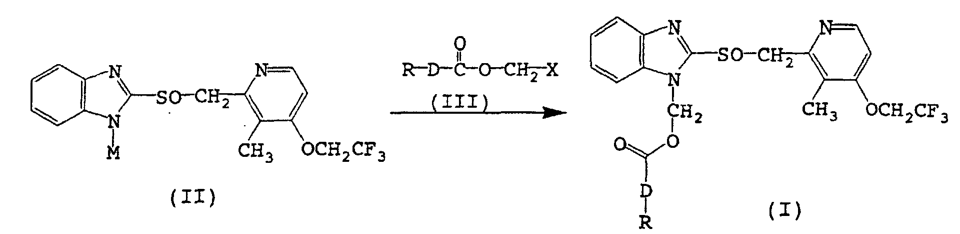 wherein M is a hydrogen atom, a metal cation or a quaternary ammonium ion, X is a halogen, and other symbols are as defined above.

For example, the Method A is performed by reacting compound (II) or a salt thereof with compound (III) in the presence of a base. To be specific, for example, abase is added to a mixed solution of compound (II) or a salt thereof and compound (III), and the mixture is stirred.

The salt of compound (II) here is exemplified by the above-mentioned salts of compound (I), which are acid addition salts such as inorganic acid salt (e.g., hydrochloride, sulfate, hydrobromide, phosphate and the like), organic acid salt (e.g., acetate, trifluoroacetate, succinate, maleate, fumarate, propionate, citrate, tartrate, lactate, oxalate, methanesulfonate, p-toluenesulfonate and the like), and the like.

The reaction of Method A is generally conducted in a solvent, and a solvent that does not inhibit the above-mentioned reaction is selected as appropriate. Examples of such solvent include alcohols (e.g., methanol, ethanol, propanol, isopropanol, butanol, tert-butanol and the like), ethers (e.g., dioxane, tetrahydrofuran, diethyl ether, tert-butyl methyl ether, diisopropyl ether, ethylene glycol dimethyl ether and the like), esters (e.g., ethyl formate, ethyl acetate, n-butyl acetate and the like), halogenated hydrocarbons (e.g., dichloromethane, chloroform, carbon tetrachloride, trichlene, 1,2-dichloroethane and the like), hydrocarbons (e.g., n-hexane, benzene, toluene and the like), amides (e.g., formamide, N,N-dimethylformamide, N,N-dimethylacetamide and the like), ketones (e.g., acetone, methyl ethyl ketone, methyl isobutyl ketone and the like), nitriles (e.g., acetonitrile, propionitrile and the like) and the like, as well as dimethyl sulfoxide, sulfolane, hexamethylphosphoramide, water and the like, which may be used alone or as a mixed solvent. The amount of the solvent to be used is not particularly limited as long as the reaction mixture can be stirred, which is generally 2- to 100-fold amount by weight of compound (II).

The base in Method A is, for example, an inorganic base such as C₁₋₆ alkyl lithium or C₆₋₁₀ aryl lithium (e.g., methyl lithium, ethyl lithium, n-butyl lithium, sec-butyl lithium, tert-butyl lithium, phenyl lithium and the like), lithium C₂₋₆ alkylamides (e.g., lithium dimethylamide, lithium diethylamide, lithium diisopropylamide and the like), metal hydrides (e.g., lithium hydride, sodium hydride and the like), alkali metal C₁₋₆ alkoxides (e.g., lithium ethoxide, lithium tert-butoxide, sodium methoxide, sodium ethoxide, potassium tert-butoxide and the like), alkali metal amides (e.g., lithium amide, potassium amide, sodium amide and the like), alkali metal hydroxide (e.g., lithium hydroxide, potassium hydroxide, sodium hydroxide and the like), carbonate or bicarbonate of alkali metal (e.g., sodium carbonate, potassium carbonate, sodium hydrogencarbonate and the like) and the like; organic base such as tertiary amine (e.g., triethylamine, tri(n-propyl)amine, tri(n-butyl)amine, diisopropylethylamine, cyclohexyldimethylamine, pyridine, lutidine, γ-collidine, N,N-dimethylaniline, N-methylpiperidine, N-methylpyrrolidine, N-methylmorpholine and the like); and the like. The amount of the base to be used is generally 1 mole-10 mole, preferably 1 mole - 3 mole, relative to 1 mole of compound (II) or a salt thereof.

In this reaction, generally 1 - 5 mole, preferably 1 - 3 mole, of compound (III) can be used per 1 mole of compound (II).

The reaction temperature is generally from about -80°C to 100°C, preferably 0°C to 60°C.

The reaction time varies depending on the kind of compounds (II) and (III) and solvent, reaction temperature and the like, but it is generally 1 min. - 72 hrs., preferably 15 min. - 24 hrs.

The α-halomethyl ester reagent represented by the formula (III) can be obtained by reacting the corresponding acyl halides with formaldehyde (L.H. Ulich and R. Adams, J. Am. Chem. Soc., vol. 43, p. 660 (1921)). For higher yield of the reaction, bromomethyl esters are more preferable.

The compound (II) can be produced according to the method described in JP-A-61-50978, USP 4,628,098 and the like or a method similar thereto.

### Method B

The compound (I) or a salt thereof can be obtained by condensing [2-[[[3-methyl-4-(2,2,2-trifluoroethoxy)-2-pyridyl]methyl]sulfinyl]-1H-benzimidazol-1-yl]methanol represented by the formula (IV) with carboxylic acid represented by the formula: R-D-COOH [the symbols in the formula are as defined above] or a reactive derivative thereof. 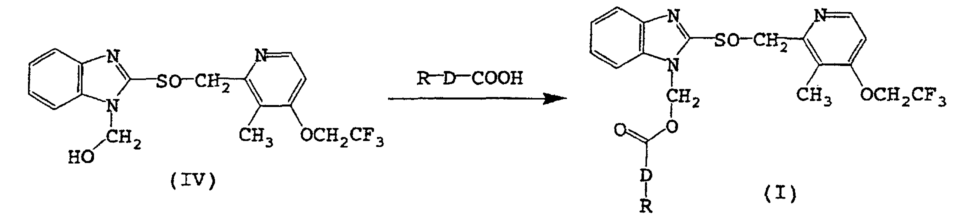 wherein each symbol is as defined above.

When a free carboxylic acid is reacted, condensation can be conducted using a condensation agent. Examples of the condensation agent include N,N'-carbodiimidazole, N,N'-dicyclohexylcarbodiimide, N,N'-diisopropylcarbodiimide, 1-ethyl-3-(3-dimethylaminopropyl)carbodiimide, N-ethoxycarbonyl-2-ethoxy-1,2-dihydroquinoline, diethyl phosphorocyanidate, diphenyl phosphoryl azide, 2-(1H-benzotriazol-1-yl)-1,1,3,3-tetramethyluronium tetrafluoroborate, 2-(1H-benzotriazol-1-yl)-1,1,3,3-tetramethyluronium hexafluorophosphate, benzotriazol-1-yl-oxy-tris(dimethylamino)phosphonium hexafluorophosphate, benzotriazol-1-yl-oxy-tris-pyrrolidino-phosphonium hexafluorophosphate, bromo-tris-pyrrolidino-phosphonium hexafluorophosphate, 2-(5-norbornene-2,3-dicarboximido)-tetramethyluronium tetrafluoroborate and the like. The amount of the condensation agent to be used is generally 1 mole - 10 mole, preferably 1 mole - 2 mole, relative to 1 mole of compound (IV) or a salt thereof.

Examples of the reactive derivative of the carboxylic acid include acid halide, acid azide, acid anhydride, mixed acid anhydride, active amide, active ester, active thioester and the like.

Examples of the above-mentioned acid halide include acid chloride, acid bromide and the like.

Examples of the mixed acid anhydride include mixed acid anhydride of mono-C₁₋₄ alkyl carbonic acid (e.g., mixed acid anhydride of the carboxylic acid with mono-methyl carbonic acid, mono-ethyl carbonic acid, mono-isopropyl carbonic acid, monoisobutyl carbonic acid, mono-tert-butyl carbonic acid, monobenzyl carbonic acid, mono(p-nitrobenzyl) carbonic acid, monoallyl carbonic acid and the like), mixed acid anhydride of C₁₋₆ aliphatic carboxylic acid (e.g., mixed acid anhydride of the carboxylic acid with acetic acid, cyanoacetic acid, propionic acid, lactic acid, isolactic acid, valeric acid, isovaleric acid, pivalic acid, trifluoroacetic acid, trichloroacetic acid, acetoacetic acid and the like), mixed acid anhydride of C₇₋₁₁ aromatic carboxylic acid (e.g., mixed acid anhydride of the carboxylic acid with benzoic acid, p-toluic acid, p-chlorobenzoic acid and the like), mixed acid anhydride of organic sulfonic acid (e.g., mixed acid anhydride of the carboxylic acid with methanesulfonic acid, ethanesulfonic acid, benzenesulfonic acid, p-toluenesulfonic acid and the like) and the like.

Examples of the active amide include amide with nitrogen-containing heterocyclic compound [e.g., acid amide of the carboxylic acid with pyrazole, imidazole, benzotriazole and the like; the nitrogen-containing heterocyclic compounds are optionally substituted by C₁₋₄ alkyl group (e.g., methyl, ethyl, propyl, isopropyl, butyl, isobutyl, sec-butyl, tert-butyl and the like), C₁₋₆ alkoxy group (e.g., methoxy, ethoxy, propoxy, isopropoxy, butoxy, tert-butoxy and the like), halogen (e.g., fluorine, chlorine, bromine and the like), oxo group, thioxo group, C₁₋₆ alkylthio group (e.g., methylthio, ethylthio, propylthio, butylthio and the like) and the like], and the like.

Examples of the active ester include organic phosphoric acid esters (e.g., diethoxyphosphoric acid ester, diphenoxyphosphoric acid ester and the like), p-nitrophenyl ester, 2;4-dinitrophenyl ester, cyanomethyl ester, pentachlorophenyl ester, N-hydroxysuccinimide ester, N-hydroxyphthalimide ester, 1-hydroxybenzotriazole ester, 6-chloro-1-hydroxybenzotriazole ester, 1-hydroxy-1H-2-pyridone ester and the like.

Examples of the active thioester include esters [e.g., 2-pyridylthiol ester, 2-benzothiazolylthiol ester] with aromatic heterocyclyl-thiol compound [the heterocycle is optionally substituted by C₁₋₄ alkyl group (e.g., methyl, ethyl, propyl, isopropyl, butyl, isobutyl, sec-butyl, tert-butyl and the like), C₁₋₆ alkoxy group (e.g., methoxy, ethoxy, propoxy, isopropoxy, butoxy, tert-butoxy and the like), halogen (e.g., fluorine, chlorine, bromine and the like), C₁₋₆ alkylthio group (e.g., methylthio, ethylthio, propylthio, butylthio and the like) and the like], and the like.

The reaction of Method B is generally conducted in a solvent, and a solvent that does not inhibit the reaction of Method B is selected as appropriate. Examples of the solvent include ethers (e.g., dioxane, tetrahydrofuran, diethyl ether, tert-butyl methyl ether, diisopropyl ether, ethylene glycol dimethyl ether and the like), esters (e.g., ethyl formate, ethyl acetate, butyl acetate and the like), halogenated hydrocarbons (e.g., dichloromethane, chloroform, carbon tetrachloride, trichlene, 1,2-dichloroethane and the like), hydrocarbons (e.g., n-hexane, benzene, toluene and the like), amides (e.g., formamide, N,N-dimethylformamide, N,N-dimethylacetamide and the like), ketones (e.g., acetone, methyl ethyl ketone, methyl isobutyl ketone and the like), nitriles (e.g., acetonitrile, propionitrile and the like) and the like, as well as dimethyl sulfoxide, sulfolane, hexamethylphosphoramide, water and the like. These may be used alone or as a mixed solvent. The amount of the solvent to be used is not particularly limited as long as the reaction mixture can be stirred, which is generally 2- to 100-fold amount by weight, preferably 5- to 50-fold amount by weight, of 1 mole of compound (IV) or a salt thereof.

The amount of carboxylic acid or a reactive derivative thereof to-be used is generally 1 mole - 10 mole, preferably 1 mole - 5 mole, relative to 1 mole of compound (IV).

The reaction of Method B is carried out in the temperature range of generally from -80°C to 200°C, preferably from -40°C to 150°C, most preferably from -30°C to 100°C.

The reaction time of Method B varies depending on the kind of carboxylic acid, a reactive derivative thereof and the solvent (and mixing ratio in the case of a mixed solvent), reaction temperature and the like, which is generally 1 min.-72 hrs., preferably 15 min. - 24 hrs.

When acid halide is used as the reactive derivative of carboxylic acid, the reaction can be carried out in the presence of a deoxidation agent to remove the eliminated hydrogen halide from the reaction system. Examples of the deoxidation agent include inorganic base (e.g., sodium carbonate, potassium carbonate, calcium carbonate, sodium hydrogencarbonate and the like), tertiary amine (e.g., triethylamine, tripropylamine, tributylamine, cyclohexyldimethylamine, pyridine, lutidine, γ-collidine, N,N-dimethylaniline, N-methylpiperidine, N-methylpyrrolidine, N-methylmorpholine, 4-dimethylaminopyridine and the like), alkylene oxides (e.g., propylene oxide, epichlorohydrin and the like) and the like. The amount of the "deoxidation agent" to be used is generally 1 mole - 10 mole, preferably 1 mole - 3 mole, relative to 1 mole of compound (IV).

The compound of the formula (IV), [2-[[[3-methyl-4-(2,2,2-trifluoroethoxy)-2-pyridyl]methyl]sulfinyl]-1H-benzimidazol-1-yl]methanol which is a starting material, can be obtained according to the means known in this field [Varma et al., Eur. J. Med. Chem., vol. 15, p. 536 (1980) and Haugwitz et al., J. Med. Chem., vol. 22, p. 1113 (1979)], by, for example, hydroxymethylation of 2-[[[3-methyl-4-(2,2,2-trifluoroethoxy)-2-pyridyl]methyl]sulfinyl]-1H-benzimidazole by a treatment with formaldehyde in a solvent (e.g., acetonitrile, ethyl acetate and the like).

### Method C

The compound (I) or a salt thereof can be obtained by subjecting compound (V) or a salt thereof to oxidation reaction. 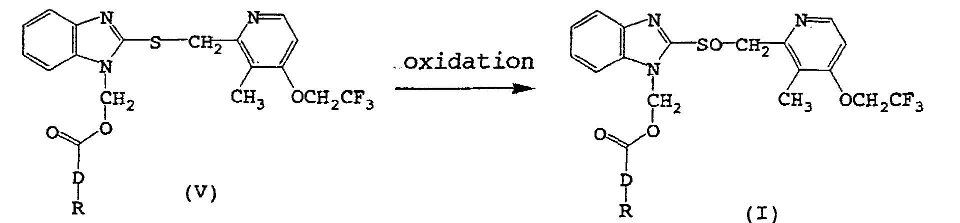 wherein each symbol is as defined above.

The reaction in Method C can be carried out using an oxidant such as nitric acid, hydrogen peroxide, peroxy acid, peroxy acid ester, ozone, dinitrogen tetraoxide, iodosobenzene, N-halosuccinimide, 1-chlorobenzotriazole, tert-butyl hypochlorite, diazabicyclo[2.2.2]octane-bromine complex, sodium metaperiodate, selenium dioxide, manganese dioxide, chromic acid, cerium ammonium nitrate, bromine, chlorine, sulfuryl chloride and the like. The amount of the oxidant to be used is generally 0.5 mole - 2 mole, preferably 0.8 mole - 1.2 mole, per 1 mole of compound (I).

The reaction of Method C is generally carried out in a solvent inert to the above-mentioned oxidation reaction. Examples of the "inert solvent" include water, alcohols (e.g., methanol, ethanol, 1-propanol, 2-propanol and the like), ketones (e.g., acetone, methyl ethyl ketone and the like), nitriles (e.g., acetonitrile, propionitrile and the like), amides (e.g., formamide, N,N-dimethylformamide and the like), ethers (e.g., diethyl ether, tert-butyl methyl ether, diisopropyl ether, dioxane, tetrahydrofuran and the like), sulfoxides (e.g., dimethyl sulfoxide and the like) and polar solvents (e.g., sulfolane, hexamethylphosphoramide and the like), which may be used alone or as a mixed solvent thereof. The "inert solvent" is used in generally 1- to 100-fold amount by weight of compound (V) or a salt thereof.

The reaction temperature is generally from -80°C to 80°C, preferably from 15°C to 30°C.

The reaction time is generally 1 min. - 6 hrs., preferably 15 min. - 1 hr.

The compound (V), which is a starting material of Method C, can be obtained by subjecting 2-[[[3-methyl-4-(2,2,2-trifluoroethoxy)-2-pyridyl]methyl]thio]-1H-benzimidazole to the reaction similar to the reaction in Method A or Method B.

The salt of compound (V) is exemplified by the above-mentioned salts of the compound (I), which are acid addition salts such as inorganic acid salt (e.g., hydrochloride, sulfate, hydrobromide, phosphate and the like), organic acid salt (e.g., acetate, trifluoroacetate, succinate, maleate, fumarate, propionate, citrate, tartrate, lactate, oxalate, methanesulfonate, p-toluenesulfonate and the like) and the like.

The compound (I) or a salt thereof obtained by the above-mentioned methods A - C can be isolated and purified from the reaction mixture by a separation means known per se (e.g., concentration, concentration under reduced pressure, solvent extraction, crystallization, recrystallization, phase transfer, chromatography and the like).

(R)-2-[[[3-Methyl-4-(2,2,2-trifluoroethoxy)-2-pyridyl]methyl]sulfinyl]-1H-benzimidazole can be produced by, for example, subjecting 2-[[[3-methyl-4-(2,2,2-trifluoroethoxy)-2-pyridyl]methyl]sulfinyl]-1H-benzimidazole or a salt thereof to optical resolution, or by asymmetric oxidation of 2-[[[3-methyl-4-(2,2,2-trifluoroethoxy)-2-pyridyl]methyl]thio]-1H-benzimidazole.

The method of optical resolution includes methods known per se, such as a fractional recrystallization method, a chiral column method, a diastereomer method, and so forth. Asymmetric oxidation includes methods known per se, such as the method described in WO96/02535 and the like.

The "fractional recrystallization method" includes a method in which a salt is formed between a racemate and an optically active compound [e.g., (+)-mandelic acid, (-)-mandelic acid, (+)-tartaric acid, (-)-tartaric acid, (+)-1-phenethylamine, (-)-1-phenethylamine, cinchonine, (-)-cinchonidine, brucine, etc.], which salt is separated by fractional recrystallization etc., and, if desired, subjected to a neutralization process to give a free optical isomer.

The "chiral column method" includes a method in which a racemate or a salt thereof is applied to a column for optical isomer separation (chiral column). In the case of liquid chromatography, for example, optical isomers are separated by adding a racemate to a chiral column such as ENANTIO-OVM (produced by Tosoh Corporation), the DAICEL CHIRAL series (produced by Daicel Corporation) and the like, and developing the racemate in water, a buffer (e.g., phosphate buffer), an organic solvent (e.g., hexane, ethanol, methanol, isopropanol, acetonitrile, trifluoroacetic acid, diethylamine, triethylamine, etc.), or a solvent mixture thereof. In the case of gas chromatography, for example, a chiral column such as CP-Chirasil-DeX CB (produced by GL Science) and the like is used to separate optical isomers.

The "diastereomer method" includes a method in which a racemate and an optically active reagent are reacted (preferably, an optically active reagent is reacted with the group at the 1-position of the benzimidazole group) to give a diastereomeric mixture, which is then subjected to ordinary separation means (e.g., fractional recrystallization, chromatography, etc.) to obtain either diastereomer, which is subjected to a chemical reaction (e.g., acid hydrolysis, base hydrolysis, hydrogenolysis, etc.) to cut off the optically active reagent moiety, whereby the desired optical isomer is obtained. Said "optically active reagent" includes, for example, optically active organic acids such as MTPA [α-methoxy-α-(trifluoromethyl)phenylacetic acid], (-)-menthoxyacetic acid and the like, optically active alkoxymethyl halides such as (1R-endo)-2-(chloromethoxy)-1,3,3-trimethylbicyclo[2.2.1]heptane and the like, and the like.

The compound (I) and a salt thereof of the present invention are useful as a pharmaceutical agent, because they have a superior anti-ulcer activity, a gastric acid secretion-suppressive action, a mucosa-protecting action, an anti-*Helicobacter pylori* action and the like, and show low toxicity. In addition, since they are stable to acid, they do not require formulation into an enteric-coated preparation for oral administration, which in turn eliminates the cost for formulating enteric-coated preparation. Moreover, the tablet can be made smaller, which is easily swallowed by patients having difficulty in swallowing, particularly the elderly and children. Furthermore, since absorption is faster than in enteric-coated preparations, expression of gastric acid secretion-suppressive action is rapid. The preparation is long-acting because it is gradually converted to the original compound in living organisms. Consequently, the compounds are useful as anti-ulcer agents and the like.

The compound (I) and a salt thereof of the present invention are useful for the prophylaxis or treatment of digestive ulcer (e.g., gastric ulcer, gastric ulcer due to post-operative stress, duodenal ulcer, anastomotic ulcer, Zollinger-Ellison syndrome, ulcer caused by nonsteroidal antiinflammatory etc.); gastritis; reflux esophagitis; NUD (Non Ulcer Dyspepsia); gastric cancer (including gastric cancer due to promoted production of interleukin-1β caused by genetic polymorphism of interleukin-1); gastric MALT lymphoma; gastric hyperacidity (e.g. gastric hyperacidity due to post-operative stress); hemorrhage of upper gastrointestinal tract caused by acute stress ulcer, hemorrhagic gastritis, invasion stress (stress due to major surgery requiring intensive management after operation and cerebrovascular disorder, external injury in the head, multiple organ failure and extensive burn requiring intensive treatment), and the like, pre-anesthetic administration, eradication of *Helicobacter pylori,* in mammals (e.g., human, simian, sheep, cattle, horse, dog, cat, rabbit, rat, mouse etc.).

The compound (I) and a salt thereof of the present invention show low toxicity and can be safely administered orally or parenterally (e.g., topical, rectal, intravenous administrations and the like) as they are or as a preparation containing a pharmaceutical composition containing a pharmacologically acceptable carrier admixed according to a method known per se, such as tablets (including sugar-coated tablets and film-coated tablets), powder, granule, capsule (including soft capsule), orally disintegrating tablet, liquid, injection, suppository, sustained-release preparation, plaster and the like.

The content of compound (I) or a salt thereof of the present invention in the pharmaceutical composition of the present invention is about 0.01 to 100% by weight relative to the entire composition. Though subject to change depending on the administration target, administration route, target disease and the like, its dose is about 0.5 to 1,500 mg/day, preferably about 5 to 150 mg/day, based on the active ingredient, when, for example, the compound is orally administered as an anti-ulcer agent to an adult human (60 kg). The compound (I) or a salt thereof of the present invention may be administered once daily or in 2 or 3 divided portions per day.

The pharmacologically acceptable carrier that may be used to produce the pharmaceutical composition of the present invention includes various organic or inorganic carrier substances in common use as pharmaceutical materials, including excipients, lubricants, binders, disintegrants, water-soluble polymers and basic inorganic salts for solid preparations; and solvents, dissolution aids, suspending agents, isotonizing agents, buffers and soothing agents for liquid preparations and the like. Other ordinary pharmaceutical additives such as preservatives, anti-oxidants, coloring agents, sweetening agents, souring agents, bubbling agents and flavorings may also be used as necessary.

Such "excipients" include, for example, lactose, sucrose, D-mannitol, starch, cornstarch, crystalline cellulose, light silicic anhydride, titanium oxide and the like.

Such "lubricants" include, for example, magnesium stearate, sucrose fatty acid esters, polyethylene glycol, talc, stearic acid and the like.

Such "binders" include, for example, hydroxypropyl cellulose, hydroxypropylmethyl cellulose, crystalline cellulose, starch, polyvinylpyrrolidone, gum arabic powder, gelatin, pullulan, low-substituted hydroxypropyl cellulose and the like.

Such "disintegrants" include (1) crosslinked povidone, (2) what is called super-disintegrants such as crosslinked carmellose sodium (FMC-Asahi Chemical) and carmellose calcium (Gotoku Yakuhin) etc, (3) carboxymethyl starch sodium (e.g., product of Matsutani Chemical), (4) low-substituted hydroxypropyl cellulose (e.g., product of Shin-Etsu Chemical), (5) corn starch, and so forth. Said "crosslinked povidone" may be any crosslinked polymer having the chemical name 1-ethenyl-2-pyrrolidinone homopolymer, including polyvinylpyrrolidone (PVPP) and 1-vinyl-2-pyrrolidinone homopolymer, and is exemplified by Colidon CL (produced by BASF), Polyplasdon XL (produced by ISP), Polyplasdon XL-10 (produced by ISP), Polyplasdon INF-10 (produced by ISP) and the like.

Such "water-soluble polymers" include, for example, ethanol-soluble water-soluble polymers [e.g., cellulose derivatives such as hydroxypropyl cellulose (hereinafter also referred to as HPC) etc, polyvinylpyrrolidone and the like], ethanol-insoluble water-soluble polymers [e.g., cellulose derivatives such as hydroxypropylmethyl cellulose (hereinafter also referred to as HPMC) etc., methyl cellulose, carboxymethyl cellulose sodium and the like, sodium polyacrylate, polyvinyl alcohol, sodium alginate, guar gum and the like] and the like.

Such "basic inorganic salts" include, for example, basic inorganic salts of sodium, potassium, magnesium and/or calcium. Preferred are basic inorganic salts of magnesium and/or calcium. More preferred are basic inorganic salts of magnesium. Such basic inorganic salts of sodium include, for example, sodium carbonate, sodium hydrogencarbonate, disodium hydrogenphosphate and the like. Such basic inorganic salts of potassium include, for example, potassium carbonate, potassium hydrogencarbonate and the like. Such basic inorganic salts of magnesium include, for example, heavy magnesium carbonate, magnesium carbonate, magnesium oxide, magnesium hydroxide, magnesium metasilicate aluminate, magnesium silicate, magnesium aluminate, synthetic hydrotalcite [Mg₆Al₂(OH)₁₆CO₃4H₂O], and alumina hydroxide magnesium. Preferred are heavy magnesium carbonate, magnesium carbonate, magnesium oxide, magnesium hydroxide and the like. Such basic inorganic salts of calcium include, for example, precipitated calcium carbonate, calcium hydroxide, etc.

Such "solvents" include, for example, water for injection, alcohol, propylene glycol, macrogol, sesame oil, corn oil, olive oil and the like.

Such "dissolution aids" include, for example, polyethylene glycol, propylene glycol, D-mannitol, benzyl benzoate, ethanol, trisaminomethane, cholesterol, triethanolamine, sodium carbonate, sodium citrate and the like.

Such "suspending agents" include, for example, surfactants such as stearyltriethanolamine, sodium lauryl sulfate, laurylaminopropionic acid, lecithin, benzalkonium chloride, benzethonium chloride, monostearic glycerol etc; hydrophilic polymers such as polyvinyl alcohol, polyvinylpyrrolidone, carboxymethyl cellulose sodium, methyl cellulose, hydroxymethyl cellulose, hydroxyethyl cellulose, hydroxypropyl cellulose etc., and the like.

Such "isotonizing agents" include, for example, glucose, D-sorbitol, sodium chloride, glycerol, D-mannitol and the like.

Such "buffers" include, for example, buffer solutions of phosphates, acetates, carbonates, citrates etc, and the like.

Such "soothing agents" include, for example, benzyl alcohol and the like.

Such "preservatives" include, for example, p-oxybenzoic acid esters, chlorobutanol, benzyl alcohol, phenethyl alcohol, dehydroacetic acid, sorbic acid and the like.

Such "antioxidants" include, for example, sulfites, ascorbic acid, α-tocopherol and the like.

Such "coloring agents" include, for example, food colors such as Food Color Yellow No. 5, Food Color Red No. 2, Food Color Blue No. 2 etc; food lake colors, red oxide and the like.

Such "sweetening agents" include, for example, saccharin sodium, dipotassium glycyrrhizinate, aspartame, stevia, thaumatin and the like.

Such "souring agents" include, for example, citric acid (citric anhydride), tartaric acid, malic acid and the like.

Such "bubbling agents" include, for example, sodium bicarbonate and the like.

Such "flavorings" may be synthetic substances or naturally occurring substances, and include, for example, lemon, lime, orange, menthol, strawberry and the like.

The compound of the present invention may be prepared as a preparation for oral administration in accordance with a commonly-known method, by, for example, compression-shaping in the presence of a carrier such as an excipient, a disintegrant, a binder, a lubricant, or the like, and subsequently coating the preparation as necessary by a commonly known method for the purpose of taste masking, enteric dissolution or sustained release. For an enteric preparation, an intermediate layer may be provided by a commonly known method between the enteric layer and the drug-containing layer for the purpose of separation of the two layers.

For preparing the compound (I) or a salt thereof of the present invention as an orally disintegrating tablet, available methods include, for example, a method in which a core containing crystalline cellulose and lactose is coated with the compound (I) or a salt thereof of the present invention and, where necessary, a basic inorganic salt, and then further coated with a coating layer containing a water-soluble polymer to give a composition, which is coated with an enteric coating layer containing polyethylene glycol, further coated with an enteric coating layer containing triethyl citrate, still further coated with an enteric coating layer containing polyethylene glycol, and finally coated with mannitol to give fine granules, which are mixed with additives and shaped.

The above-mentioned "enteric coating layer" includes, for example, a layer consisting of a mixture of one or more kinds from aqueous enteric polymer substrates such as cellulose acetate phthalate (CAP), hydroxypropylmethyl cellulose phthalate, hydroxymethyl cellulose acetate succinate, methacrylic acid copolymers (e.g., Eudragit L30D-55 (trade name; produced by Rohm), Colicoat MAE30DP (trade name; produced by BASF), Polyquid PA30 (trade name; produced by San-yo Chemical) etc), carboxymethylethyl cellulose, shellac and the like; sustained-release substrates such as methacrylic acid copolymers (e.g., Eudragit NE30D (trade name), Eudragit RL30D (trade name), Eudragit RS30D (trade name), etc.) and the like; water-soluble polymers; plasticizers such as triethyl citrate, polyethylene glycol, acetylated monoglycerides, triacetin, castor oil and the like; and the like, and the like.

The above-mentioned "additive" includes, for example, water-soluble sugar alcohols (e.g., sorbitol, mannitol, maltitol, reduced starch saccharides, xylitol, reduced palatinose, erythritol, etc.), crystalline cellulose (e.g., Ceolas KG 801, Avicel PH 101, Avicel PH 102, Avicel PH 301, Avicel PH 302, Avicel RC-591 (crystalline cellulose carmellose sodium) etc), low-substituted hydroxypropyl cellulose (e.g., LH-22, LH-32, LH-23, LH-33 (Shin-Etsu Chemical), mixtures thereof etc) and the like. Furthermore, binders, souring agents, bubbling agents, sweetening agents, flavorings, lubricants, coloring agents, stabilizers, excipients, disintegrants etc. are also used.

The compound of the present invention may be used in combination with 1 to 3 other active ingredients.

Such "other active ingredients" include, for example, anti-*Helicobacter pylori* active substances, imidazole compounds, bismuth salts, quinolone compounds, and so forth. Of these substances, preferred are anti-*Helicobacter pylori* active substances, imidazole compounds etc.

Such "anti-*Helicobacter pylori* active substances" include, for example, antibiotic penicillins (e.g., amoxicillin, benzylpenicillin, piperacillin, mecillinam, etc.), antibiotic cefems (e.g., cefixime, cefaclor, etc.), antibiotic macrolides (e.g., erythromycin, clarithromycin, etc.), antibiotic tetracyclines (e.g., tetracycline, minocycline, streptomycin, etc.), antibiotic aminoglycosides (e.g., gentamicin, amikacin, etc.), imipenem and so forth. Of these substances, preferred are antibiotic penicillins, antibiotic macrolides and the like.

Such "imidazole compounds" include, for example, metronidazole, miconazole and the like.

Such "bismuth salts" include, for example, bismuth acetate, bismuth citrate and the like.

Such "quinolone compounds" include, for example, ofloxacin, ciploxacin and the like.

For eradication of *Helicobacter pylori,* a combination therapy of compound (I) or a salt thereof of the present invention with antibiotic penicillin (e.g., amoxicillin and the like) and antibiotic erythromycin (e.g., clarithromycin and the like) is particularly preferable.

Such "other active ingredients" and the compound (I) or a salt thereof of the present invention may be mixed, prepared as a single pharmaceutical composition [e.g., tablets, powders, granules, capsules (including soft capsules), liquids, injectable preparations, suppositories, sustained-release preparations, etc.], in accordance with a commonly known method, and used in combination, and may also be prepared as separate preparations and administered to the same subject simultaneously or at a time interval.

### Examples

The present invention is explained in more detail by referring to Reference Examples and Examples, which are not to be construed as limitative.

In the following Reference Example and Examples, the term "room temperature" indicates about 15 to 30°C.

¹H-NMR spectra were determined with CDCl₃ as the solvent using Varian Gemini-200 and Mercury-300; data are shown in chemical shift δ(ppm) from the internal standard tetramethylsilane.

Optical rotation [α]_{D} was determined at 20°C, using the DIP-370 Digital polarimeter (produced by JASCO).

The enantiomer excess (%ee) was measured by high performance liquid chromatography using an optically active column under the following conditions.
high performance liquid chromatography conditions (A);
column: CHIRALCEL OD (manufactured by Daicel Chemical Industries, Ltd.)
mobile phase: hexane/ethanol=90/10
flow rate: 1.0 mL/min.
detection: UV 285 nm
The other symbols used herein have the following definitions:
s: singlet
d: doublet
t: triplet
q: quartet
m: multiplet
bs: broad singlet
J: binding constant

### Reference Example 1

### (R)-2-[[[3-Methyl-4-(2,2,2-trifluoroethoxy)-2-pyridinyl]methyl]sulfinyl]-1H-benzimidazole

2-[[[3-Methyl-4-(2,2,2-trifluoroethoxy)-2-pyridinyl]methyl]thio]-1H-benzimidazole (4.5 kg, 12.7 mol, containing water 1.89 g), toluene (22 L), water (25 g, 1.39 mol, 1.49 mol as total water content) and (+)-diethyl tartrate (0.958 L, 5.60 mol) were mixed under a nitrogen stream. Titanium(IV) isopropoxide (0.747 L, 2.53 mol) was added to the mixture at 50 - 60°C under a nitrogen stream, and the mixture was stirred at the same temperature for 30 min. Diisopropylethylamine (0.733 L, 4.44 mol) was added to the obtained mixture at room temperature under a nitrogen stream, and cumene hydroperoxide (6.88 L, content 82%, 37.5 mol) was added at -5°C to 5°C. The mixture was stirred at -5°C to 5°C for 1.5 hrs. to give a reaction mixture. 30% Aqueous sodium thiosulfate solution (17 L) was added to the reaction mixture under a nitrogen stream, and the remaining cumene hydroperoxide was decomposed. After partitioning, water (4.5 L), heptane (13.5 L), t-butyl methyl ether (18 L) and heptane (27 L) were added successively to the obtained organic layer, and crystals were precipitated under stirring. The crystals were separated and washed with t-butyl methyl ether-toluene (t-butyl methyl ether:toluene=4:1)(4 L). A suspension of the wet crystal in acetone (20 L) was added dropwise with stirring to a mixture of acetone (7 L) and water (34 L), and water (47 L) was added. The precipitated crystals were separated and washed with acetone-water (acetone:water=1:3)(4 L) and water (12 L). The wet crystals were dissolved in ethyl acetate (45 L) and water (3 L), and the solution was partitioned. The trace amount of the insoluble material in the organic layer was filtered off, and triethylamine (0.2 L) was added. The mixture was concentrated under reduced pressure until the liquid amount became about 7 L. Methanol (2.3 L), ca. 12.5% aqueous ammonia (23 L, ca. 50°C) and t-butyl methyl ether (22 L, ca. 50°C) were added to the concentrate, and the mixture was partitioned. To the organic layer was added ca. 12.5% aqueous ammonia (11 L), and the mixture was partitioned (this step was repeated once). The aqueous layers were combined, and ethyl acetate (22 L) was added. Acetic acid was added dropwise under cooling, and pH was adjusted to about 8. After partitioning, the aqueous layer was extracted with ethyl acetate (11 L). The organic layers were combined and washed with ca. 20% brine (11 L). After addition of triethylamine (0.2 L), the organic layer was concentrated under reduced pressure. Acetone (5 L) was added to the concentrate, and the mixture was concentrated under reduced pressure. The concentrate was dissolved in acetone (9 L), and the solution was added dropwise to a mixture of acetone (4.5 L) and water (22.5 L), and water (18 L) was added dropwise to the obtained mixture. The precipitated crystals were separated and washed successively with cooled acetone-water (acetone:water=1:3)(3 L) and water (12 L). The wet crystals were dissolved in ethyl acetate (32 L). The separated aqueous layer was separated by partitioning, and the obtained organic layer was concentrated under reduced pressure until the liquid amount became about 14 L. Ethyl acetate (36 L) and activated carbon (270 g) were added to the residual liquid and, after stirring, the activated carbon was filtered off. The filtrate was concentrated under reduced pressure until the liquid amount became about 14 L. Heptane (90 L) was added dropwise to the remaining liquid at about 40°C. After stirring at the same temperature for about 30 min., the crystals were separated and washed with ethyl acetate - heptane (ethyl acetate:heptane=1:8, 6 L, ca. 40°C). Drying gave the title compound (3.4 kg). The enantiomer excess of this compound was 100% ee.

### Reference Example 2

### [2-[[[3-Methyl-4-(2,2,2-trifluoroethoxy)-2-pyridinyl]methyl]sulfinyl]-1H-benzimidazol-1-yl]methanol

To a mixture of 2-[[[3-methyl-4-(2,2,2-trifluoroethoxy)-2-pyridinyl]methyl]sulfinyl]-1H-benzimidazole (3.69 g), dichloromethane (50 mL) and 4-dimethylaminopyridine (0.12 g) was added a solution of 37% formaldehyde solution (3.75 mL) diluted with water (6.25 mL) at room temperature. This mixture was stirred vigorously at room temperature for 8 min, and the dichloromethane layer was separated and dried over sodium sulfate. After filtration, concentration under reduced pressure gave the title compound (3.99 g) as an amorphous compound.
¹H-NMR(CDCl₃): 2.34(3H,s), 4.38(2H,q,J=7.8Hz), 4.98(2H,s), 5.76(1H,d,J=11.3Hz), 6.12(1H,d,J=11.3Hz), 6.60(1H,d,J=5.8Hz), 7.29-7.47(2H,m), 7.59(1H,d,J=7.8Hz), 7.74(1H,d,J=7.6Hz), 8.10(1H,d,J=5.8Hz).

### Reference Example 3

### [(R)-2-[[[3-Methyl-4-(2,2,2-trifluoroethoxy)-2-pyridinyl]methyl]sulfinyl]-1H-benzimidazol-1-yl]methanol

To a solution of (R)-2-[[[3-methyl-4-(2,2,2-trifluoroethoxy)-2-pyridinyl]methyl]sulfinyl]-1H-benzimidazole (1.0 g) in dichloromethane (10 mL) was added a solution of 4-dimethylaminopyridine (33 mg) and 37% aqueous formalin solution (1.5 mL) diluted with water (2 mL), and the mixture was stirred at room temperature for 1 hr. The reaction mixture was extracted with dichloromethane-water, and the organic layer was washed with saturated brine, dried over anhydrous sodium sulfate and concentrated under reduced pressure. The residue was crystallized from dichloromethane-isopropyl ether to give the title compound as a pale-brown solid (0.75 g).
¹H-NMR(CDCl₃): 2.40(3H,s), 4.39(2H,q,J=8.0Hz), 4.98(2H,s), 5.75(1H,d,J=11.4Hz), 6.12(1H,d,J=11.4Hz), 6.60(1H,d,J=5.8Hz), 7.30-7.50(2H,m), 7.59(1H,dd,J=1.2,7.8Hz),
7.74(1H,dd,J=1.0,7.6Hz), 8.10(1H,d,J=5.8Hz)

### Example 1

### [2-[[[3-Methyl-4-(2,2,2-trifluoroethoxy)-2-pyridinyl]methyl]sulfinyl]-1H-benzimidazol-1-yl]methyl benzoate

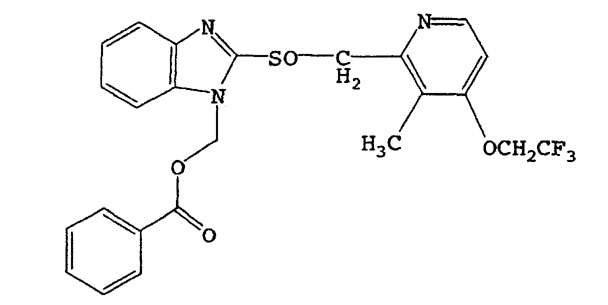

To a solution (10 mL) of [2-[[[3-methyl-4-(2,2,2-trifluoroethoxy)-2-pyridinyl]methyl]sulfinyl]-1H-benzimidazol-1-yl]methanol (1.99 g) in tetrahydrofuran were added triethylamine (1.4 mL) and benzoyl chloride (0.7 mL) under ice-cooling. The mixture was stirred under ice-cooling for 4.5 hrs. Ethyl acetate (60 mL) was added, and the mixture was washed with water (30 mL) and saturated brine (15 mL), and dried over anhydrous magnesium sulfate. After concentration under reduced pressure, diethyl ether (30 mL) was added to the residue to allow solidification, which was collected by filtration and washed with diethyl ether. The title compound (1.13 g) was obtained as a colorless solid by drying under reduced pressure.
¹H-NMR(CDCl₃): 2.29(3H,s), 4.37(2H,q,J=7.8Hz), 5.03(1H,d,J=13.8Hz), 5.11(1H,d,J=13.8Hz), 6.63(1H,d,J=5.7Hz), 6.76(2H,s), 7.33-7.50(4H,m), 7.58(1H,m), 7.73-7.85(2H,m), 8.03-8.10(2H,m), 8.28(1H,d,J=5.7Hz)

### Example 2

### [2-[[[3-Methyl-4-(2,2,2-trifluoroethoxy)-2-pyridinyl]methyl]sulfinyl]-1H-benzimidazol-1-yl]methyl trimethylacetate

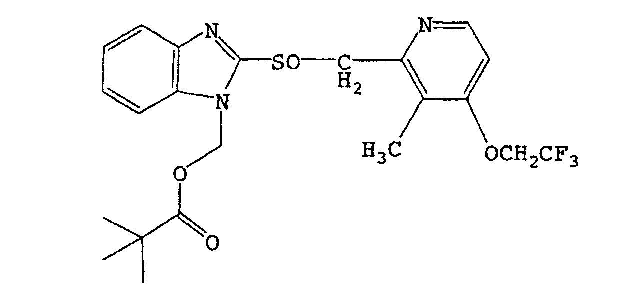

To a solution (25 mL) of [2-[[[3-methyl-4-(2,2,2-trifluoroethoxy)-2-pyridinyl]methyl]sulfinyl]-1H-benzimidazol-1-yl]methanol (1.99 g) in tetrahydrofuran were added triethylamine (1.4 mL) and trimethylacetyl chloride (0.924 mL) under ice-cooling. The mixture was stirred under ice-cooling for 2.5 hrs. Ethyl acetate (60 mL) was added, and the mixture was washed with water (30 mL), aqueous sodium hydrogencarbonate (30 mL) and saturated brine (30 mL) and dried over anhydrous magnesium sulfate. After concentration under reduced pressure, a mixed solution of diethyl ether and diisopropyl ether was added to the residue to allow solidification, which was collected by filtration. The solid (1.06 g) obtained by drying under reduced pressure was washed with ethyl acetate-diisopropyl ether and dried under reduced pressure to give the title compound (0.432 g) as a colorless solid.
¹H-NMR(CDCl₃): 1.18(9H,s), 2.30(3H,s), 4.38(2H,q,J=7.8Hz), 4.99(1H,d,J=13.7Hz), 5.07(1H,d,J=13.7Hz) 6.48(1H,d,J=11.3Hz), 6.55(1H,d,J=11.3Hz), 6.65(1H,d,J=5.7Hz), 7.32-7.48(2H,m), 7.62(1H,m), 7.82(1H,m), 8.29(1H,d,J=5.7Hz)

### Example 3

### [2-[[[3-Methyl-4-(2,2,2-trifluoroethoxy)-2-pyridinyl]methyl]sulfinyl]-1H-benzimidazol-1-yl]methyl acetate

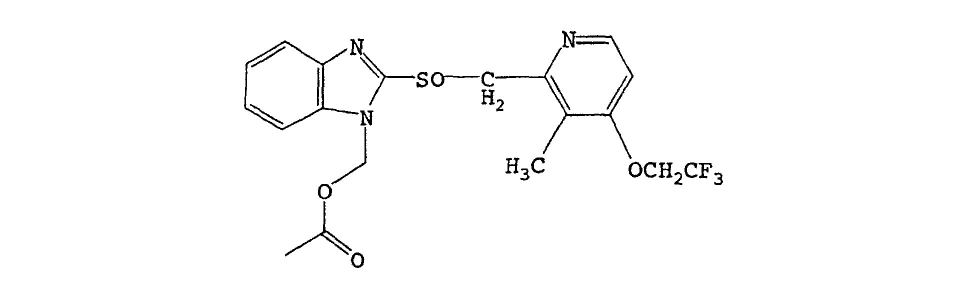

To a solution (10 mL) of [2-[[[3-methyl-4-(2,2,2-trifluoroethoxy)-2-pyridinyl]methyl]sulfinyl]-1H-benzimidazol-1-yl]methanol (1.99 g) in tetrahydrofuran were added triethylamine (1.4 mL) and acetyl chloride (0.354 mL) under ice-cooling. The mixture was stirred under ice-cooling for 2.5 hrs. Ethyl acetate (60 mL) was added, and the mixture was washed with water (30 mL) and saturated brine (10 mL) and dried over anhydrous magnesium sulfate. The mixture was concentrated under reduced pressure, and diethyl ether (30 mL) was added to the residue to allow solidification, which was collected by filtration and washed with diethyl ether. The title compound (1.46 g) was obtained as a colorless solid by drying under reduced pressure.
¹H-NMR(CDCl₃): 2.12(3H,s), 2.31(3H,s), 4.39(2H,q,J=8.0Hz), 4.96(1H,d,J=13.8Hz), 5.07(1H,d,J=13.8Hz), 6.51(2H,s), 6.65(1H,d,J=5.6Hz), 7.33-7.48(2H,m), 7.64(1H,m), 7.82(1H,m) 8.29(1H,d,J=5.6Hz)

### Example 4

### [2-[[[3-Methyl-4-(2,2,2-trifluoroethoxy)-2-pyridinyl]methyl]sulfinyl]-1H-benzimidazol-1-yl]methyl phenylacetate

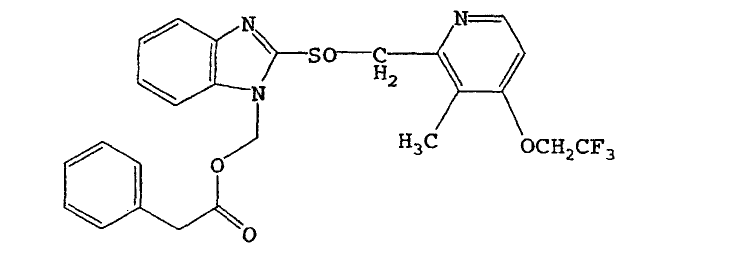

To a solution (40 mL) of [2-[[[3-methyl-4-(2,2,2-trifluoroethoxy)-2-pyridinyl]methyl]sulfinyl]-1H-benzimidazol-1-yl]methanol (1.99 g) in tetrahydrofuran were added triethylamine (1.4 mL) and phenylacetyl chloride (0.8 mL) under ice-cooling. The mixture was stirred under ice-cooling for 9 hrs. Ethyl acetate (60 mL) was added, and the mixture was washed with water (30 mL), aqueous sodium hydrogencarbonate (30 mL) and saturated brine (30 mL) and dried over anhydrous magnesium sulfate. The mixture was concentrated under reduced pressure, and the residue was purified by silica gel column chromatography (eluted with ethyl acetate:hexane=1:1, then with ethyl acetate). The obtained solid was washed with diethyl ether and dried under reduced pressure to give the title compound (1.28 g) as a colorless solid.
¹H-NMR(CDCl₃): 2.28(3H,s), 3.68(2H,s), 4.38(2H,q,J=7.9Hz), 4.90(1H,d,J=13.8Hz), 5.00(1H,d,J=13.8Hz), 6.49(1H,d,J=11.0Hz), 6.56(1H,d,J=11.0Hz), 6.63(1H,d,J=5.5Hz), 7.15-7.30(5H,m), 7.31-7.44(2H,m), 7.58(1H,m), 7.80(1H,m), 8.26(1H,d,J=5.5Hz)

### Example 5

### (R)-[2-[[[3-Methyl-4-(2,2,2-trifluoroethoxy)-2-pyridinyl]methyl]sulfinyl]-1H-benzimidazol-1-yl]methyl benzoate

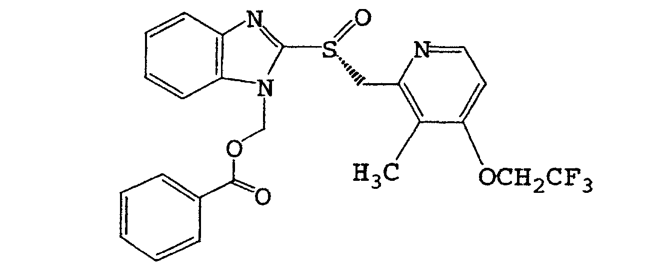

To a mixture of (R)-2-[[[3-methyl-4-(2,2,2-trifluoroethoxy)-2-pyridinyl]methyl]sulfinyl]-1H-benzimidazole (3.69 g), ethyl acetate (50 mL) and 4-dimethylaminopyridine (0.12 g) was added a 37% formaldehyde solution (3.76 mL) at room temperature. This mixture was vigorously stirred at room temperature for 1 hr., and the reaction mixture was washed with water (10 mL) and dried over anhydrous magnesium sulfate. The residue was treated with activated carbon and, after filtration, concentrated under reduced pressure. The obtained residue was dissolved in tetrahydrofuran (10 mL), and triethylamine (1.5 mL) and benzoyl chloride (1.27 mL) were added under ice-cooling. The mixture was stirred under ice-cooling for 1 hr. Ethyl acetate (50 mL) was added, and the mixture was washed with water (30 mL), aqueous sodium hydrogencarbonate (30 mL) and saturated brine (15 mL) and dried over anhydrous magnesium sulfate. The residue was treated with activated carbon and filtrated, and the filtrate was concentrated under reduced pressure. The residue was purified by silica gel column chromatography (eluted with ethyl acetate:hexane=1:1, then with ethyl acetate). The obtained solid was recrystallized from ethyl acetate-hexane. Then, recrystallization from tetrahydrofuran-diethyl ether gave the title compound (1.60 g) as a colorless solid.
¹H-NMR(CDCl₃): 2.30(3H,s), 4.37(2H,q,J=7.9Hz), 5.03(1H,d,J=14.2Hz), 5.10(1H,d,J=14.2Hz), 6.63(1H,d,J=5.6Hz), 6.76(2H,s), 7.34-7.49(4H,m), 7.54-7.61(1H,m), 7.74-7.86(2H,m), 8.03-8.08(2H,m), 8.28(1H,d,J=5.6Hz)
[α]D=+105.3° (c=1.002% in CHCl₃)

### Example 6

### [2-[[[3-Methyl-4-(2,2,2-trifluoroethoxy)-2-pyridinyl]methyl]sulfinyl]-1H-benzimidazol-1-yl]methyl 4-methylbenzoate

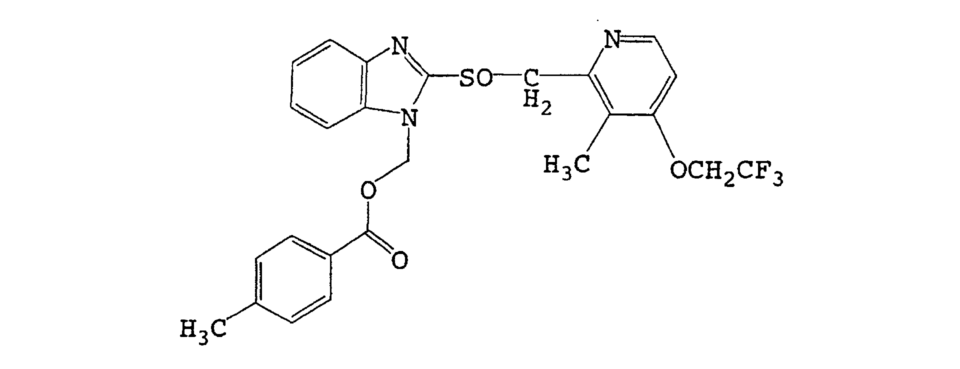

To a solution (25 mL) of [2-[[[3-methyl-4-(2,2,2-trifluoroethoxy)-2-pyridinyl]methyl]sulfinyl]-1H-benzimidazol-1-yl]methanol (1.99 g) in tetrahydrofuran were added triethylamine (1.4 mL) and 4-methylbenzoyl chloride (0.8 mL) under ice-cooling. The mixture was stirred under ice-cooling for 3 hrs. Ethyl acetate (60 mL) was added, and the mixture was washed with water (30 mL), aqueous sodium hydrogencarbonate (30 mL) and saturated brine (30 mL) and dried over anhydrous magnesium sulfate. The mixture was concentrated under reduced pressure, and the obtained solid was washed with diethyl ether and dried under reduced pressure. The residue was washed with ethyl acetate-diisopropyl ether and dried under reduced pressure to give the title compound (1.04 g) as a colorless solid.
¹H-NMR(CDCl₃): 2.29(3H,s), 2.39(3H,s), 4.37(2H,q,J=7.8Hz), 5.01(1H,d,J=13.6Hz), 5.10(1H,d,J=13.6Hz), 6.63(1H,d,J=5.7Hz), 6.74(2H,s), 7.21(2H,d,J=8.0Hz), 7.33-7.48(2H,m), 7.75(1H,d,J=7.0Hz), 7.83(1H,d,J=8.8Hz), 7.94(2H,d,J=8.0Hz) 8.29(1H,d,J=5.7Hz)

### Example 7

### Benzyl [2-[[[3-methyl-4-(2,2,2-trifluoroethoxy)-2-pyridinyl]methyl]sulfinyl]-1H-benzimidazol-1-yl]methyl carbonate

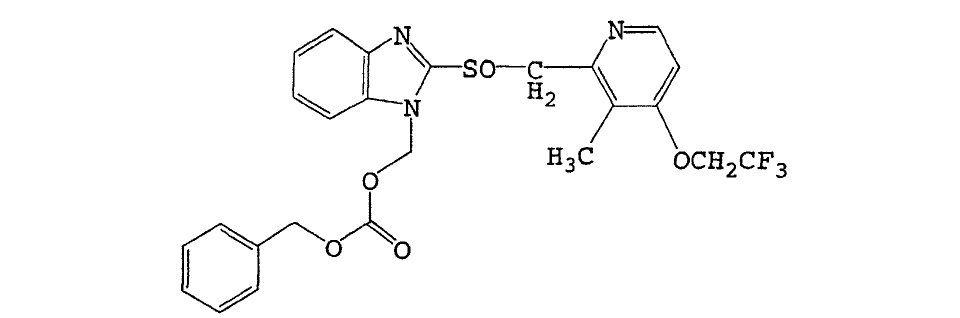

To a solution (20 mL) of [2-[[[3-methyl-4-(2,2,2-trifluoroethoxy)-2-pyridinyl]methyl]sulfinyl]-1H-benzimidazol-1-yl]methanol (1.99 g) in tetrahydrofuran were added triethylamine (1.4 mL) and benzyl chloroformate (0.86 mL). The mixture was stirred at room temperature for 3 hrs. Benzyl chloroformate (0.60 mL) was added, and the mixture was stirred at room temperature for 14.5 hrs. Ethyl acetate (150 mL) was added, and the mixture was washed with water (100 mL) and saturated brine (30 mL) and dried over anhydrous magnesium sulfate. The mixture was concentrated under reduced pressure, and the obtained solid was washed with diisopropyl ether and dried under reduced pressure to give the title compound (2.37 g) as a colorless solid.
¹H-NMR(CDCl₃): 2.25(3H,s), 4.35(2H,q,J=7.8Hz), 4.93(1H,d,J=13.8Hz), 5.06(1H,d,J=13.8Hz), 5.18(2H,s), 6.49(1H,d,J=11.2Hz), 6.61(1H,d,J=5.7Hz), 6.64(1H,d,J=11.2Hz), 7.34(5H,s), 7.34-7.49(2H,m), 7.66(1H,m), 7.82(1H,m), 8.28(1H,d,J=5.7Hz)

### Example 8

### Ethyl [2-[[[3-methyl-4-(2,2,2-trifluoroethoxy)-2-pyridinyl]methyl]sulfinyl]-1H-benzimidazol-1-yl]methyl carbonate

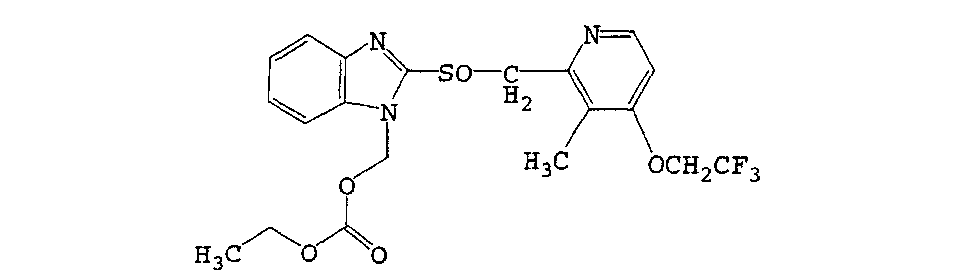

To a solution (20 mL) of [2-[[[3-methyl-4-(2,2,2-trifluoroethoxy)-2-pyridinyl]methyl]sulfinyl]-1H-benzimidazol-1-yl]methanol (1.99 g) in tetrahydrofuran were added triethylamine (1.4 mL) and ethyl chlorocarbonate (0.60 mL). The mixture was stirred at room temperature for 1 hr. Ethyl chlorocarbonate (0.40 mL) was added, and the mixture was stirred at room temperature for 16 hrs. Ethyl acetate (50 mL) was added, and the mixture was washed with water (60 mL). The aqueous layer was extracted with ethyl acetate (30 mL), and the ethyl acetate layers were combined, washed with saturated brine (20 mL) and dried over anhydrous magnesium sulfate. After concentration under reduced pressure, the residue was purified by silica gel column chromatography (eluted with ethyl acetate:hexane=1:3-1:2). The obtained solid was washed with diisopropyl ether and dried under reduced pressure to give the title compound (1.01 g) as a colorless solid.
¹H-NMR(CDCl₃): 1.29(3H,t,J=7.2Hz), 2.29(3H,s), 4.23(2H,q,J=7.2Hz), 4.38(2H,q,J=7.8Hz), 4.93(1H,d,J=13.7Hz), 5.08(1H,d,J=13.7Hz), 6.49(1H,d,J=11.1Hz), 6.61(1H,d,J=11.1Hz), 6.65(1H,d,J=5.5Hz), 7.33-7.48(2H,m)7.68(1H,m), 7.82(1H,m), 8.31(1H,d,J=5.5Hz)

### Example 9

### [(R)-2-[[[3-Methyl-4-(2,2,2-trifluoroethoxy)-2-pyridinyl]methyl]sulfinyl]-1H-benzimidazol-1-yl]methyl trimethylacetate

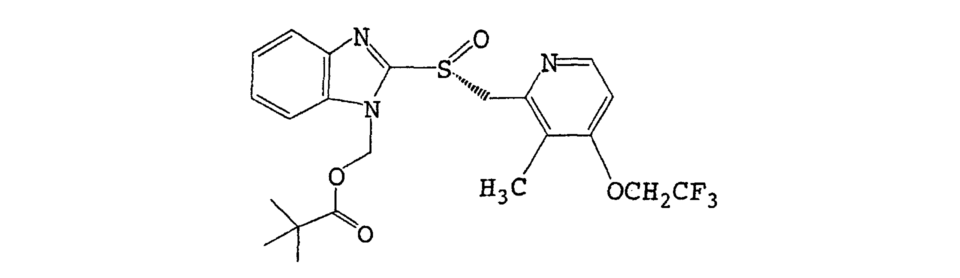

To a solution (15 mL) of (R)-2-[[[3-methyl-4-(2,2,2-trifluoroethoxy)-2-pyridinyl]methyl]sulfinyl]-1H-benzimidazole (1.5 g) in dichloromethane were added 4-dimethylaminopyridine (49.6 mg) and a solution of 37% aqueous formalin solution (1.5 mL) diluted with water (1.5 mL). The mixture was stirred at room temperature for 5 min. The reaction mixture was extracted with dichloromethane-water, and the organic layer was washed with saturated brine, dried over anhydrous sodium sulfate and concentrated under reduced pressure. The residue was dissolved in tetrahydrofuran (20 mL), and pivaloyl chloride (0.6 mL) and triethylamine (0.68 mL) were added. The mixture was stirred at room temperature for 1 hr., and the reaction mixture was extracted with ethyl acetate-water. The organic layer was washed with saturated brine, dried over anhydrous sodium sulfate and concentrated under reduced pressure. The residue was purified by silica gel column chromatography (eluted with ethyl acetate:hexane=2:1), and the purified product was crystallized from ethyl acetate-isopropyl ether to give the title compound as a colorless solid (0.32 g).
¹H-NMR(CDCl₃): 1.18(9H,s), 2.31(3H,s), 4.38(2H,q,J=7.8Hz), 4.98(1H,d,J=13.6Hz), 5.07(1H,d,J=13.6Hz), 6.50(1H,d,J=11.2Hz), 6.54(1H,d,J=11.2Hz), 6.64(1H,d,J=6.0Hz), 7.30-7.50(2H,m), 7.60-7.70(1H,m), 7.80-7.90(1H,m), 8.29(1H,d,J=6.0Hz)

### Example 10

### Ethyl [(R)-2-[[[3-methyl-4-(2,2,2-trifluoroethoxy)-2-pyridinyl]methyl]sulfinyl]-1H-benzimidazol-1-yl]methyl carbonate

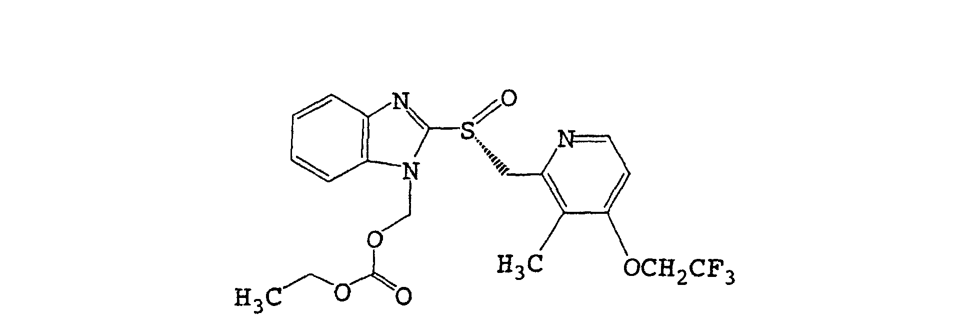

To a solution (20 mL) of [(R)-2-[[[3-methyl-4-(2,2,2-trifluoroethoxy)-2-pyridinyl]methyl]sulfinyl]-1H-benzimidazol-1-yl]methanol (2.16 g) obtained in Reference Example 3 in dichloromethane were added triethylamine (1.13 mL) and ethyl chloroformate (0.669 mL) under ice-cooling, and the mixture was stirred for 1 hr. The reaction mixture was extracted with dichloromethane-water, and the organic layer was washed with saturated brine, dried over anhydrous sodium sulfate and concentrated under reduced pressure. The residue was recrystallized from ethyl acetate to give the title compound as a colorless solid (1.0 g).
¹H-NMR(CDCl₃): 1.29(3H,t,J=7.8Hz), 2.29(3H,s), 4.23(2H,q,J=7.0Hz), 4.38(2H,q,J=7.8Hz), 4.93(1H,d,J=14.0Hz), 5.07(1H,d,J=14.0Hz), 6.49(1H,d,J=11.4Hz), 6.64(1H,d,J=11.4Hz), 6.66(1H,d,J=5.4Hz), 7.30-7.50(2H,m), 7.68(1H,dd,J=1.4,7.8Hz), 7.82(1H,dd,J=1.4,8.2Hz), 8.30(1H,d,J=5.4Hz)

### Example 11

### Benzyl [(R)-2-[[[3-methyl-4-(2,2,2-trifluoroethoxy)-2-pyridinyl]methyl]sulfinyl]-1H-benzimidazol-1-yl]methyl carbonate

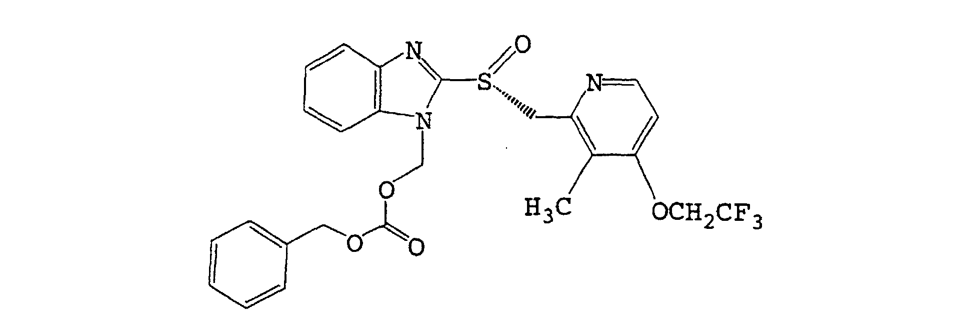

Using [(R)-2-[[[3-methyl-4-(2,2,2-trifluoroethoxy)-2-pyridinyl]methyl]sulfinyl]-1H-benzimidazol-1-yl]methanol (2.16 g) obtained in Reference Example 3 and benzyl chloroformate (0.999 mL), a similar process as in Example 10 was conducted to give the title compound as a colorless solid (1.08 g).
¹H-NMR(CDCl₃): 2.25(3H,s), 4.35(2H,q,J=8.2Hz), 4.93(1H,d,J=13.8Hz 5.05(1H,d,J=13.8Hz) 5.18(2H,s), 6.48(1H,d,J=11.0Hz), 6.59(1H,d,J=5.8Hz) 6.63(1H,d,J=11.0Hz), 7.30-7.50(7H,m), 7.66(1H,dd,J=1.4,7.4Hz), 7.81(1H,dd,J=2.0,7.0Hz), 8.27(1H,d,J=5.8Hz)

### Example 12

### Isopropyl [(R)-2-[[[3-methyl-4-(2,2,2-trifluoroethoxy)-2-pyridinyl]methyl]sulfinyl]-1H-benzimidazol-1-yl]methyl carbonate

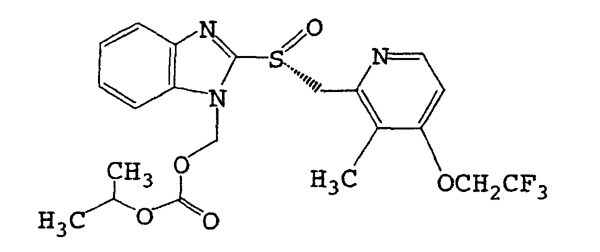

Using [(R)-2-[[[3-methyl-4-(2,2,2-trifluoroethoxy)-2-pyridinyl]methyl]sulfinyl]-1H-benzimidazol-1-yl]methanol (2.16 g) obtained in Reference Example 3 and isopropyl chloroformate (0.858 mg), a similar process as in Example 10 was conducted to give the title compound as a colorless solid (0.75 g).
¹H-NMR(CDCl₃): 1.20-1.40(6H,m), 2.28(3H,s), 4.38(2H,q,J=8.0Hz), 4.80-5.00(1H,m), 4.97(1H,d,J=13.6Hz), 5.07(1H,d,J=13.6Hz), 6.48(1H,d,J=11.4Hz), 6.60(1H,d,J=11.4Hz), 6.63(1H,d,J=6.0Hz), 7.30-7.50(2H,m), 7.68(1H,d,J=7-4Hz), 7.82(1H,dd,J=1.4,6.6Hz), 8.30(1H,d,J=6.0Hz)

### Example 13

### Butyl [(R)-2-[[[3-methyl-4-(2,2,2-trifluoroethoxy)-2-pyridinyl]methyl]sulfinyl]-1H-benzimidazol-1-yl]methyl carbonate

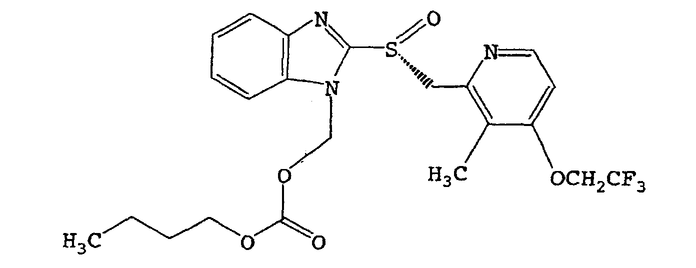

Using [(R)-2-[[[3-methyl-4-(2,2,2-trifluoroethoxy)-2-pyridinyl]methyl]sulfinyl]-1H-benzimidazol-1-yl]methanol (2.16 g) obtained in Reference Example 3 and butyl chloroformate (0.894 mL), a similar process as in Example 10 was conducted to give the title compound as a colorless solid (1.37 g).
¹H-NMR(CDCl₃): 0.90(3H,t,J=7.4Hz), 1.20-1.50(2H,m), 1.55-1.75(2H,m), 2.29(3H,s), 4.17(2H,t,J=6.6Hz), 4.38(2H,q,J=7.8Hz), 4.93(1H,d,J=13.8Hz), 5.07(1H,d,J=13.8Hz), 6.49(1H,d,J=11.4Hz), 6.64(1H,d,J=11.4Hz), 6.65(1H,d,J=5.6Hz), 7.30-7.50(2H,m), 7.68(1H,dd,J=2.0,7.0Hz), 7.82(1H,dd,J=1.4,7.0Hz), 8.31(1H,d,J=5.6Hz)

### Example 14

### [2-[[[3-Methyl-4-(2,2,2-trifluoroethoxy)-2-pyridinyl]methyl]sulfinyl]-1H-benzimidazol-1-yl]methyl 4-tert-butylbenzoate

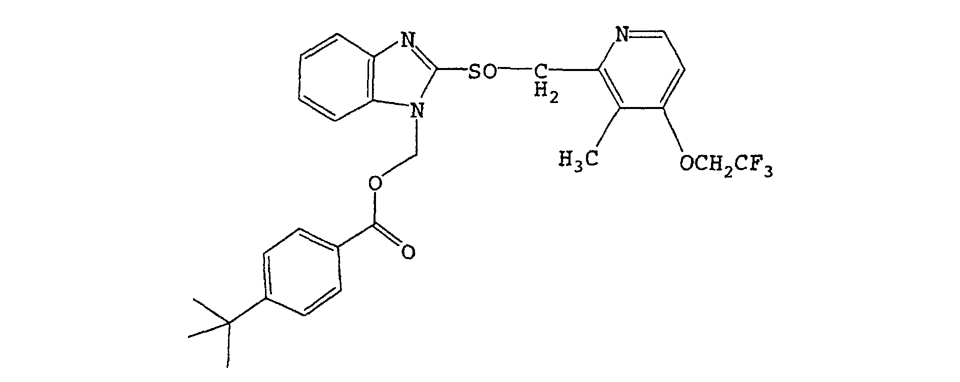

To a solution (10 mL) of [2-[[[3-methyl-4-(2,2,2-trifluoroethoxy)-2-pyridinyl]methyl]sulfinyl]-1H-benzimidazol-1-yl]methanol (0.5 g) obtained in Reference Example 2 in tetrahydrofuran were added triethylamine (0.348 mL) and 4-tert-butylbenzoyl chloride (271 mg) under ice-cooling, and the mixture was stirred at room temperature for 3 hrs. The reaction mixture was extracted with ethyl acetate-water, and the organic layer was washed with saturated brine, dried over anhydrous sodium sulfate and concentrated under reduced pressure. The residue was recrystallized from ethyl acetate to give the title compound as a colorless solid (0.282 g).
¹H-NMR(CDCl₃): 1.30(9H,s), 2.29(3H,s), 4.37(2H,q,J=8.0Hz), 5.01(1H,d,J=14.2Hz 5.09(1H,d,J=14.2Hz), 6.63(1H,d,J=5.4Hz), 6.75(2H,s), 7.30-7.50(4H,m), 7.70-7.90(2H,m), 7.98(2H,d,J=8.4Hz), 8.29(1H,d,J=5.4Hz)

### Example 15

### 2-Methoxyethyl [2-[[[3-methyl-4-(2,2,2-trifluoroethoxy)-2-pyridinyl]methyl]sulfinyl]-1H-benzimidazol-1-yl]methyl carbonate

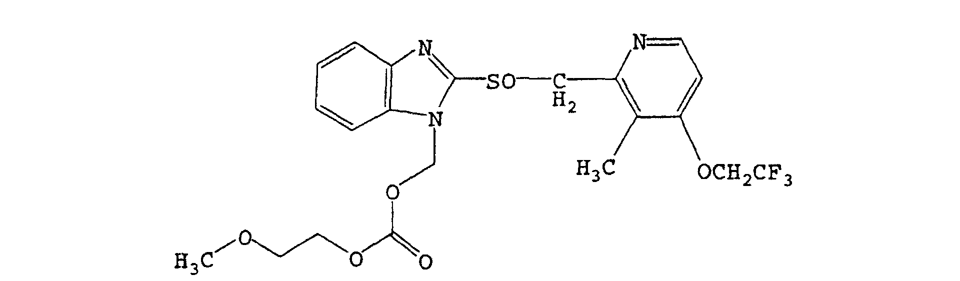

To a solution (10 mL) of [2-[[[3-methyl-4-(2,2,2-trifluoroethoxy)-2-pyridinyl]methyl]sulfinyl]-1H-benzimidazol-1-yl]methanol (1.0 g) obtained in Reference Example 2 in tetrahydrofuran were added triethylamine (0.533 mL) and 2-methoxyethyl chloroformate (0.346 mL) under ice-cooling, and the mixture was stirred at 0°C for 1 hr. The reaction mixture was extracted with ethyl acetate-water, and the organic layer was washed with saturated brine, dried over anhydrous sodium sulfate and concentrated under reduced pressure. The residue was recrystallized from ethyl acetate to give the title compound as a colorless solid (0.20 g).
¹H-NMR(CDCl₃): 2.28(3H,s), 3.34(3H,s), 3.58(2H,t,J=4.8Hz), 4.31(2H,t,J=4.8Hz), 4.38(2H,q,J=7.0Hz), 4.94(1H,d,J=13.8Hz), 5.06(1H,d,J=13.8Hz), 6.49(1H,d,J=11.4Hz), 6.63(1H,d,J=11.4Hz), 6.64(1H,d,J=5.8Hz), 7.30-7.50(2H,m), 7.66(1H,dd,J=1.4,7.4Hz), 7.82(1H,dd,J=1.4,7.4Hz), 8.30(1H,d,J=5.8Hz)

### Example 16

### [2-[[[3-Methyl-4-(2,2,2-trifluoroethoxy)-2-pyridinyl]methyl]sulfinyl]-1H-benzimidazol-1-yl]methyl isobutyrate

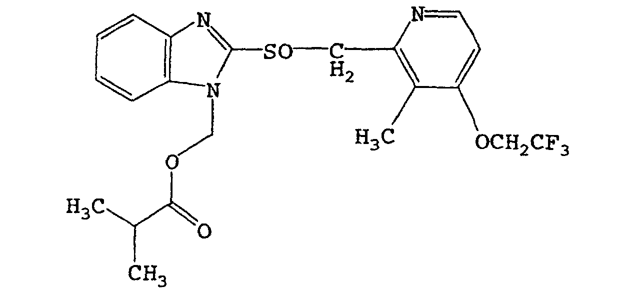

To a solution (15 mL) of [2-[[[3-methyl-4-(2,2,2-trifluoroethoxy)-2-pyridinyl]methyl]sulfinyl]-1H-benzimidazol-1-yl]methanol (2.16 g) obtained in Reference Example 2 in dichloromethane were added triethylamine (1.13 mL) and isobutyryl chloride (0.731 mL) under ice-cooling, and the mixture was stirred at 0°C for 1.5 hrs. The reaction mixture was extracted with dichloromethane-water, and the organic layer was washed with saturated brine, dried over anhydrous sodium sulfate and concentrated under reduced pressure. The residue was purified by silica gel column chromatography (ethyl acetate), and the purified product was recrystallized from ethyl acetate to give the title compound as a colorless solid (0.90 g).
¹H-NMR(CDCl₃): 1.10-1.20(6H,m), 2.30(3H,s), 2.50-2.70(1H,m), 4.38(2H,q,J=7.8Hz), 4.98(1H,d,J=13.6Hz), 5.07(1H,d,J=13.6Hz), 6.52(2H,s), 6.65(1H,d,J=6.0Hz) 7.30-7.50(2H,m), 7.63(1H,dd,J=2.2,7.4Hz), 7.81(1H,dd,J=1.4,7.0Hz), 8.28(1H,d,J=6.0Hz)

### Example 17

### [2-[[[3-Methyl-4-(2,2,2-trifluoroethoxy)-2-pyridinyl]methyl]sulfinyl]-1H-benzimidazol-1-yl]methyl (acetylamino)acetate

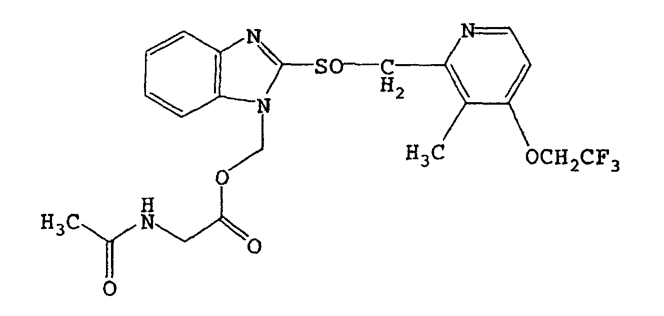

To a solution (15 mL) of N-acetylglycine (1.89 g) in N,N-dimethylformamide was added N,N'-dicyclohexylcarbodiimide (1.67 g), and the mixture was stirred at room temperature for 1 hr. The reaction mixture was filtered, and the filtrate was added to a solution (15 mL) of [2-[[[3-methyl-4-(2,2,2-trifluoroethoxy)-2-pyridinyl]methyl]sulfinyl]-1H-benzimidazol-1-yl]methanol (2.16 g) obtained in Reference Example 2 and triethylamine (1.88 mL) in dichloromethane under ice-cooling, and the mixture was stirred at 0°C for 1.5 hrs. The reaction mixture was extracted with ethyl acetate-water, and the organic layer was washed with saturated brine, dried over anhydrous sodium sulfate and concentrated under reduced pressure. The residue was recrystallized from ethyl acetate to give the title compound as a colorless solid (1.50 g).
¹H-NMR(CDCl₃): 1.99(3H,s), 2.33(3H,s), 4.00-4.25(2H,m), 4.39(2H,q,J=7.8Hz), 5.02(2H,s), 6.30-6.40(1H,bs), 6.51(1H,d,J=11.0Hz), 6.59(1H,d,J=11.0Hz), 6.64(1H,d,J=5.4Hz), 7.30-7.50(2H,m), 7.62(1H,d,J=7.4Hz), 7.79(1H,dd,J=1.6,6.6Hz), 8.22(1H,d,J=5.4Hz)

### Example 18

### [2-[[[3-Methyl-4-(2,2,2-trifluoroethoxy)-2-pyridinyl]methyl]sulfinyl]-1H-benzimidazol-1-yl]methyl propyl carbonate

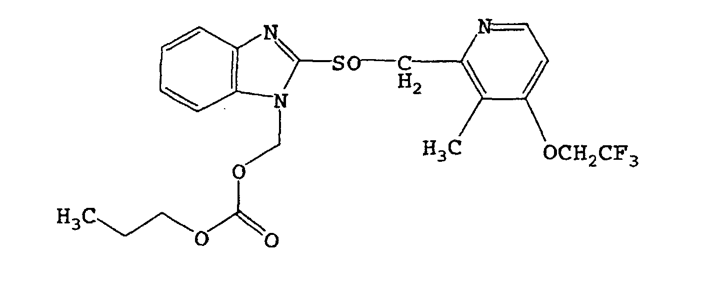

To a solution (15 mL) of [2-[[[3-methyl-4-(2,2,2-trifluoroethoxy)-2-pyridinyl]methyl]sulfinyl]-1H-benzimidazol-1-yl]methanol (2.16 g) obtained in Reference Example 2 in dichloromethane were added triethylamine (1.13 mL) and propyl chloroformate (0.787 mL) under ice-cooling, and the mixture was stirred at 0°C for 1.5 hrs. The reaction mixture was extracted with dichloromethane-water, and the organic layer was washed with saturated brine, dried over anhydrous sodium sulfate and concentrated under reduced pressure. The residue was recrystallized from ethyl acetate to give the title compound as a colorless solid (1.10 g).
¹H-NMR(CDCl₃): 0.92(3H,t,J=7.6Hz), 1.55-1.80(2H,m), 2.29(3H,s), 4.13(2H,t,J=6.6Hz), 4.38(2H,q,J=7.8Hz), 4.93(1H,d,J=13.8Hz), 5.08(1H,d,J=13.8Hz) 6.48(1H,d,J=11.0Hz), 6.61(1H,d,J=11.0Hz), 6.63(1H,d,J=5.8Hz), 7.30-7.50(2H,m), 7.68(1H,dd,J=1.4,7.8Hz), 7.82(1H,dd,J=1.8,8.4Hz), 8.31(1H,d,J=5.8Hz)

### Example 19

### Isopropyl [2-[[[3-methyl-4-(2,2,2-trifluoroethoxy)-2-pyridinyl]methyl]sulfinyl]-1H-benzimidazol-1-yl]methyl carbonate

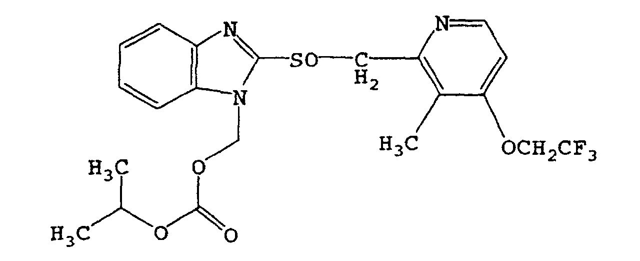

Using [2-[[[3-methyl-4-(2,2,2-trifluoroethoxy)-2-pyridinyl]methyl]sulfinyl]-1H-benzimidazol-1-yl]methanol (1.0 g) obtained in Reference Example 2 and isopropyl chloroformate (0.47 g), a similar process as in Example 10 was conducted to give the title compound as a colorless solid (0.50 g).
¹H-NMR(CDCl₃): 1.20-1.35(6H,m), 2.28(3H,s), 4.38(2H,q,J=7.8Hz), 4.80-5.00(1H,m), 4.93(1H,d,J=13.8Hz), 5.08(1H,d,J=13.8Hz), 6.48(1H,d,J=11.0Hz), 6.60(1H,d,J=11.0Hz), 6.64(1H,d,J=5.4Hz), 7.30-7.50(2H,m), 7.68(1H,dd,J=1.8,7.0Hz),
7.82(1H,dd,J=1.4,6.6Hz), 8.32(1H,d,J=5.4Hz)

### Example 20

### Butyl [2-[[[3-methyl-4-(2,2,2-trifluoroethoxy)-2-pyridinyl]methyl]sulfinyl]-1H-benzimidazol-1-yl]methyl carbonate

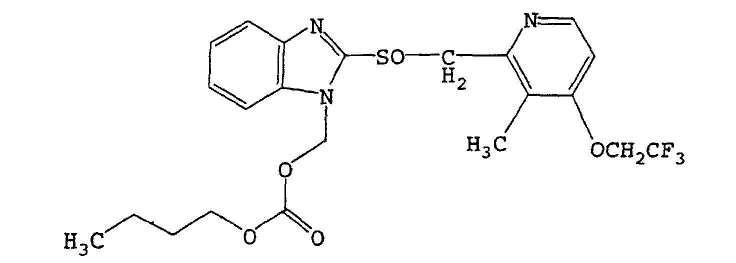

To a mixture of 2-[[[3-methyl-4-(2,2,2-trifluoroethoxy)-2-pyridinyl]methyl]sulfinyl]-1H-benzimidazole (739 mg), dichloromethane (10 mL) and 4-dimethylaminopyridine (24 mg) was added a 37% formaldehyde solution (0.745 mL) at room temperature. This mixture was stirred vigorously at room temperature for 30 min., and water (5 mL) was added. The dichloromethane layer was separated and dried over sodium sulfate. After filtration, triethylamine (0.558 mL) and butyl chloroformate (0.382 mL) were added under ice-cooling. The mixture was stirred under ice-cooling for 5 hrs. The reaction mixture was concentrated under reduced pressure, and ethyl acetate (50 mL) was added. The mixture was washed with aqueous sodium hydrogencarbonate (30 mL), dried over anhydrous magnesium sulfate and concentrated under reduced pressure. The residue was dissolved in methanol (50 mL), and aqueous sodium hydrogencarbonate (1 mL) was added. The mixture was stirred for 0.5 hr. The solvent was concentrated under reduced pressure, and ethyl acetate (50 mL) was added. The mixture was washed with water (30 mL), dried over anhydrous magnesium sulfate and concentrated under reduced pressure. The residue was purified by silica gel column chromatography (eluted with ethyl acetate:hexane=1:1 and then with ethyl acetate). The obtained solid was washed with a mixed solution of ethyl acetate and diisopropyl ether and dried under reduced pressure to give the title compound (421 mg) as a colorless solid.
¹H-NMR(CDCl₃): 0.90(3H,t,J=7.4Hz), 1.28-1.42(2H,m), 1.57-1.68(2H,m), 2.29(3H,s), 4.17(2H,t,J=6.8Hz), 4.38(2H,q,J=7.8Hz), 4.93(1H,d,J=13.8Hz), 5.08(1H,d,J=13.8Hz), 6.49(1H,d,J=11.3Hz), 6.61(1H,d,J=11.3Hz) 6.64(1H,d,J=5.9Hz), 7.34-7.48(2H,m), 7.65-7.70(1H,m), 7.80-7.85(1H,m), 8.31(1H,d,J=5.9Hz).

### Example 21

### [(R)-2-[[[3-Methyl-4-(2,2,2-trifluoroethoxy)-2-pyridinyl]methyl]sulfinyl]-1H-benzimidazol-1-yl]methyl acetate

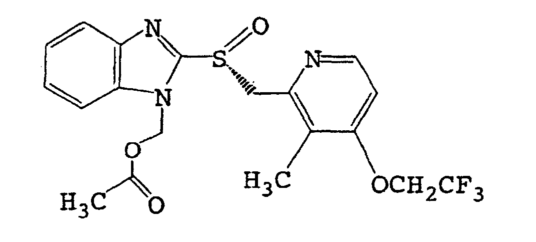

Using [(R)-2-[[[3-methyl-4-(2,2,2-trifluoroethoxy)-2-pyridinyl]methyl]sulfinyl]-1H-benzimidazol-1-yl]methanol (2.16 g) obtained in Reference Example 3 and acetyl chloride (0.498 mL), a similar process as in Example 10 was conducted to give the title compound as a colorless solid (0.87 g).
¹H-NMR(CDCl₃): 2.13(3H,s), 2.31(3H,s), 4.39(2H,q,J=8.0Hz), 4.96(1H,d,J=13.8Hz) 5.07(1H,d,J=13.8Hz), 6.51(2H,s), 6.65(1H,d,J=5.6Hz), 7.30-7.50(2H,m), 7.65(1H,dd,J=1.4,6.6Hz), 7.82(1H,dd,J=2.2,7.0Hz), 8.29(1H,d,J=5.6Hz).

### Example 22

### Propyl [(R)-2-[[[3-methyl-4-(2,2,2-trifluoroethoxy)-2-pyridinyl]methyl]sulfinyl]-1H-benzimidazol-1-yl]methyl carbonate

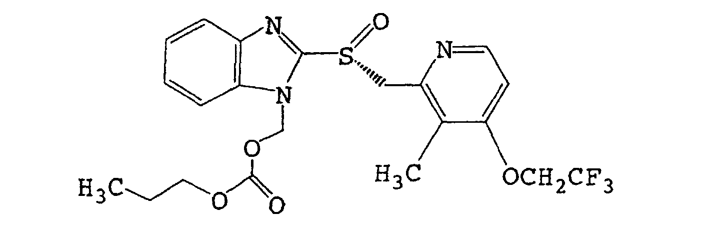

Using [(R)-2-[[[3-methyl-4-(2,2,2-trifluoroethoxy)-2-pyridinyl]methyl]sulfinyl]-1H-benzimidazol-1-yl]methanol (2.16 g) obtained in Reference Example 3 and propyl chloroformate (0.787 mL), a similar process as in Example 10 was conducted to give the title compound as a colorless solid (0.89 g).
¹H-NMR(CDCl₃): 0.92(3H,t,J=7.2Hz), 1.50-1.80(2H,m), 2.29(3H,s), 4.12(2H,t,J=6.2Hz), 4.38(2H,q,J=7.6Hz), 4.93(1H,d,J=14.0Hz), 5.09(1H,d,J=14.0Hz), 6.49(1H,d,J=11.4Hz), 6.61(1H,d,J=11.4Hz), 6.64(1H,d,J=5.4Hz), 7.30-7.50(2H,m), 7.68(1H,dd,J=1.4,8.8Hz), 7.82(1H,dd,J=1.6,6.6Hz), 8.30(1H,d,J=5.4Hz).

### Example 23

### [(R)-2-[[[3-Methyl-4-(2,2,2-trifluoroethoxy)-2-pyridinyl]methyl]sulfinyl]-1H-benzimidazol-1-yl]methyl isobutyrate

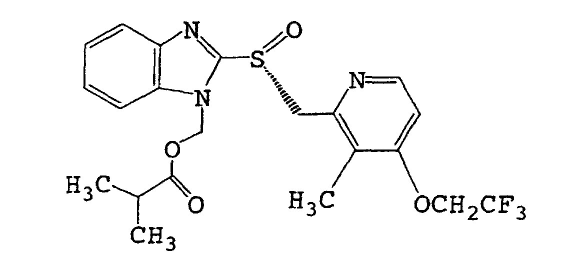

Using [(R)-2-[[[3-methyl-4-(2,2,2-trifluoroethoxy)-2-pyridinyl]methyl]sulfinyl]-1H-benzimidazol-1-yl]methanol (2.16 g) obtained in Reference Example 3 and isobutyryl chloride (0.731 mL), a similar process as in Example 10 was conducted to give the title compound as a colorless solid (0.87 g).
¹H-NMR(CDCl₃): 1.10-1.20(6H,m), 2.31(3H,s), 2.50-2.70(1H,m), 4.38(2H,q,J=8.1Hz), 4.99(1H,d,J=14.1Hz), 5.06(1H,d,J=14.1Hz), 6.50(1H,d,J=11.1Hz), 6.54(1H,d,J=11.1Hz), 6.64(1H,d,J=5.4Hz), 7.30-7.50(2H,m), 7.63(1H,d,J=8.1Hz), 7.82(1H,d,J=6.9Hz), 8.29(1H,d,J=5.4Hz).

### Example 24

### [(R)-2-[[[3-Methyl-4-(2,2,2-trifluoroethoxy)-2-pyridinyl]methyl]sulfinyl]-1H-benzimidazol-1-yl]methyl propirate

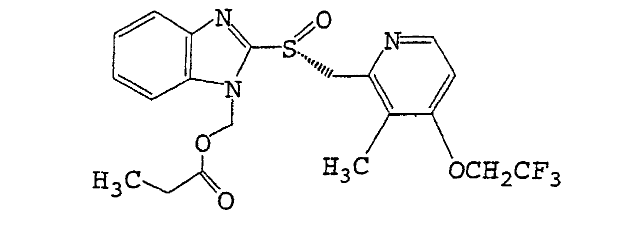

Using [(R)-2-[[[3-methyl-4-(2,2,2-trifluoroethoxy)-2-pyridinyl]methyl]sulfinyl]-1H-benzimidazol-1-yl]methanol (2.16 g) obtained in Reference Example 3 and propanoyl chloride (0.608 mL), a similar process as in Example 10 was conducted to give the title compound as a colorless solid (0.89 g).
¹H-NHR(CDCl₃): 1.13(3H,t,J=7.5Hz), 2.31(3H,s), 2.39(2H,q,J=7.5Hz), 4.38(2H,q,J=7.8Hz), 4.97(1H,d,J=13.8Hz), 5.06(1H,d,J=13.8Hz), 6.50(1H,d,J=11.4Hz), 6.54(1H,d,J=11.4Hz), 6.64(1H,d,J=5.7Hz), 7.30-7.50(2H,m), 7.65(1H,dt,J=1.2,8.7Hz), 7.81(1H,dt,J=1.0,7.8Hz), 8.28(1H,d,J=5.7Hz).

### Example 25

### 2-Methoxyethyl [(R)-2-[[[3-methyl-4-(2,2,2-trifluoroethoxy)-2-pyridinyl]methyl]sulfinyl]-1H-benzimidazol-1-yl]methyl carbonate

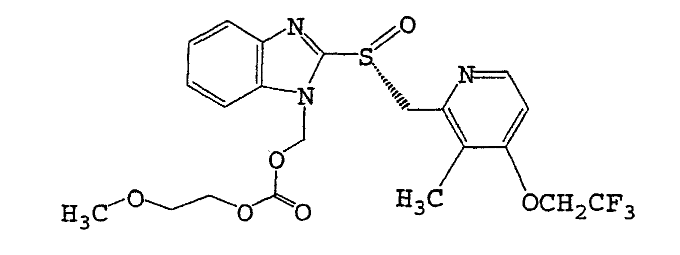

Using [(R)-2-[[[3-methyl-4-(2,2,2-trifluoroethoxy)-2-pyridinyl]methyl]sulfinyl]-1H-benzimidazol-1-yl]methanol (2.16 g) obtained in Reference Example 3 and 2-methoxyethyl chloroformate (0.970 mL), a similar process as in Example 10 was conducted to give the title compound as a colorless solid (1.00 g).
¹H-NMR(CDCl₃): 2.28(3H,s), 3.34(3H,s), 3.55-3.60(2H,m), 4.29-4.35(2H,m), 4.38(2H,q,J=7.8Hz), 4.95(1H,d,J=13.8Hz), 5.06(1H,d,J=13.8Hz), 6.49(1H,d,J=11.1Hz), 6.62(1H,d,J=11.1Hz), 6.64(1H,d,J=5.4Hz), 7.30-7.50(2H,m), 7.66(1H,dd,J=1.5,7.8Hz), 7.82(1H,dd,J=1.5,7.5Hz), 8.30(1H,d,J=5.4Hz).

### Experimental Example 1

### Stability test in artificial gastric juice

2-[[[3-Methyl-4-(2,2,2-trifluoroethoxy)-2-pyridyl]methyl]sulfinyl]-1H-benzimidazole(lansoprazole) and the compounds (ca. 1 - 2 mg) of Examples 1, 2 and 18 were respectively dissolved in acetonitrile (1 mL). To each solution (0.2 mL) were added acetonitrile (1 mL) and artificial gastric juice (3 mL, a solution prepared by dissolving sodium chloride (2.0 g) in hydrochloric acid (7.0 mL) and adding water to the amount of 1000 mL, pH ca. 1.2), and the stability of each compound after preservation at 37°C was examined. Samples were taken immediately after addition of acetonitrile and artificial gastric juice, and 4, 8, 12, 16 and 20 hours thereafter, analyzed under the following HPLC analysis conditions, and the half-life was calculated on the assumption that the decomposition of the compound was a pseudo-first-order reaction. The results are shown in Table 1.
HPLC analysis conditions
detection wavelength: UV 285 nm
column: YMC Pro C18 75 mm × 4.6 mm I.D.
mobile phase: the proportion of mobile phase A (0.01 mol/L aqueous ammonium acetate solution) and mobile phase B (acetonitrile) was changed with the lapse of time as in the following.
10 min. from immediately after addition: mobile phase
A/mobile phase B = 60/40→10/90
10 min. to 15 min.: mobile phase A/mobile phase B = 10/90
15.1 min. to 20 min.: mobile phase A/mobile phase B = 60/40
flow rate: 1.0 mL/min.
column temperature: 25°C
feed amount: 10 µL

**Table 1**

| Stability of compound in artificial gastric juice | |
|---|---|
| Compound | half-life (hr) |
| lansoprazole | <0.03 |
| compound of Example 1 | 4.8 |
| compound of Example 2 | 13.8 |
| compound of Example 18 | 6.4 |

The half-life of lansoprazole was less than 0.03 hr., and lansoprazole was quickly decomposed by the artificial gastric juice. In contrast, the compounds of Examples 1, 2 and 18 showed a half-life of not less than 4 hrs., and were stable to acid. Therefore, it is clear that an enteric-coated preparation does not need to be formulated when the compound of the present invention is orally administered.

### Experimental Example 2

### Experiment of conversion to lansoprazole using liver S9 and small intestine S9

Various S9s shown in Table 2 were prepared in 50 mM phosphate buffer (pH 7.4) to a final concentration of 1 mg protein/mL. To this solution (99 µL) were added 1 µL of 1 mM solution of each compound (compounds of Examples 1 and 2), wherein the 1 mM solution was prepared by dissolving each compound in methanol containing dimethyl sulfoxide in ca. 20% to give a 10 mM solution and diluting 10-fold with methanol. The mixture was incubated (reacted) at 37°C. The reaction was quenched by adding acetonitrile (100 µL) immediately after the start of the incubation and 5 min thereafter. After quenching, the reaction mixture was subjected to centrifugal separation (2500 rpm × 10 min), and the obtained supernatant was applied to HPLC. The HPLC analysis conditions then are shown in the following.

**Table 2**

| | Animal species | Maker | Product | Lot |
|---|---|---|---|---|
| Liver S9 | IGS/SD rat (male) | XenoTech | R4000.S9 | 0010147 |
| Liver S9 | human | XenoTech | H0610.S9 | 063099A |
| Small intestine S9 | IGS/SD rat (male) | XenoTech | R1000.IS9 | 0110077 |
| Small intestine S9 | human | XenoTech | H0610.IS9 | 0110085 |

HPLC analysis conditions
detection wavelength: UV 280 nm
column: CAPCELL PAK C18 MG (4.6 mm I.D. × 75 mm)
mobile phase: the proportion of mobile phase A (0.01 mol/L aqueous ammonium acetate solution) and mobile phase B (acetonitrile) was changed with the lapse of time as in the following.
7 min. from immediately after addition: mobile phase A/mobile phase B = 60/40→10/90
7 min. to 10 min.: mobile phase A/mobile phase B = 10/90
10 min. to 15 min.: mobile phase A/mobile phase B = 60/40
flow rate: 1 mL/min.
column temperature: 40°C
feed amount: 50 µL

### Result treatment

With regard to the lansoprazole (LPZ) peak area, LPZ production rate was calculated based on the area of non-converted compound immediately after the start of incubation as 100% (absorption rate unamended).

**Table 3**

| Conversion to lansoprazole by liver S9 and small intestine S9 | | | |
|---|---|---|---|
| Compound | Animal species | Liver S9 | Small intestine S9 |
| | | LPZ production (%) | LPZ production (%) |
| compound of Example 1 | rat | 60.9 | 63.6 |
| | human | 80.3 | 97.4 |
| compound of Example 2 | rat | 64.4 | 51.9 |
| | human | 88.6 | 100.0 |

From Table 3, the treatment of the compound of the present invention with rat and human liver S9 and small intestine S9 led to the rapid conversion to lansoprazole. Thus, the compound of the present invention is suggested to function as a prodrug of lansoprazole.

### Industrial Applicability

The compound of the present invention is converted to lansoprazole, which is a proton pump inhibitor, in living organisms and shows a superior anti-ulcer activity, a gastric acid secretion-suppressive action, a mucosa-protecting action, an anti-*Helicobacter pylori* action and the like. In addition, the compound shows low toxicity and is useful as a pharmaceutical product. Moreover, since the compound is stable to acid, it does not need to be formulated into an enteric-coated preparation, thereby eliminating the cost for formulating enteric-coated preparation. In addition, since the tablet becomes small, it is easy to take for patients with weak swallowing capability, particularly the elderly and children. Furthermore, since absorption is faster than in enteric-coated preparations, expression of a gastric acid secretion-suppressive action is rapid. The preparation is sustainable because it is gradually converted to a conventionally-known proton pump inhibitor in living organisms. Consequently, the compound is useful as anti-ulcer agent and the like.

This application is based on a patent application No. 316864/2000 filed in Japan, the contents of which are all hereby incorporated by reference.

## Claims

1. A benzimidazole compound represented by the formula (I) 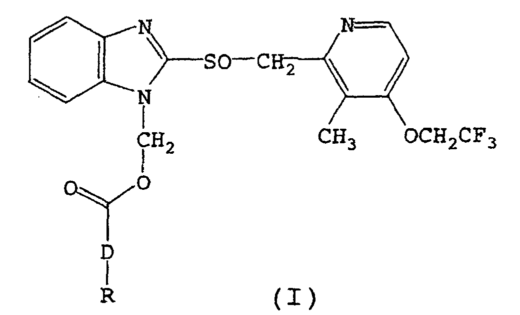 wherein D is an oxygen atom or a bond, and R is a hydrocarbon group optionally having substituents, or a salt thereof.

2. The compound of claim 1, wherein R is a C₁₋₆ alkyl group, a C₂₋₆ alkenyl group or a C₂₋₆ alkynyl group, which optionally has substituents selected from the group consisting of (i) a C₆₋₁₄ aryl group, (ii) a hydroxyl group, (iii) a halogen, (iv) a C₁₋₆ alkoxy group optionally substituted by a halogen, (v) a C₇₋₁₂ aralkyloxy group, (vi) a C₁₋₅ alkoxy-carbonyl group and (vii) an acylamino group, or a C₃₋₈ cycloalkyl group or a C₆₋₁₄ aryl group, which optionally has substituents selected from the group consisting of (i) a C₆₋₁₄ aryl group, (ii) a hydroxyl group, (iii) a halogen, (iv) a C₁₋₆ alkoxy group optionally substituted by a halogen, (v) a C₇₋₁₂ aralkyloxy group, (vi) a C₁₋₅ alkoxy-carbonyl group and (vii) a C₁₋₆ alkyl group optionally substituted by a halogen, or a salt thereof.

3. The compound of claim 1, wherein R is a C₁₋₆ alkyl group optionally having substituents selected from the group consisting of (i) a C₆₋₁₄ aryl group, (ii) a hydroxyl group, (iii) a halogen and (iv) a C₁₋₆ alkoxy group optionally substituted by a halogen, (v) a C₇₋₁₂ aralkyloxy group, (vi) a C₁₋₅ alkoxy-carbonyl group and (vii) an acylamino group, or a C₃₋₈ cycloalkyl group or a C₆₋₁₄ aryl group, which optionally has substituents selected from the group consisting of (i) a C₆₋₁₄ aryl group, (ii) a hydroxyl group, (iii) a halogen, (iv) a C₁₋₆ alkoxy group optionally substituted by a halogen, (v) a C₇₋₁₂ aralkyloxy group, (vi) a C₁₋₅ alkoxy-carbonyl group and (vii) a C₁₋₆ alkyl group optionally substituted by a halogen, or a salt thereof.

4. The compound of claim 1, wherein D is a bond and R is an alkyl group optionally having substituents or an aryl group optionally having substituents, or a salt thereof.

5. The compound of claim 4, wherein R is (1) a C₁₋₆ alkyl group optionally having 1 to 5 substituents selected from the group consisting of (i) a C₆₋₁₄ aryl group, (ii) a hydroxyl group, (iii) a halogen, (iv) a C₁₋₆ alkoxy group optionally substituted by 1 to 5 halogens, (v) a C₇₋₁₂ aralkyloxy group and (vi) a C₁₋₅ alkoxy-carbonyl group, or (2) a C₆₋₁₄ aryl group optionally having 1 to 5 substituents selected from the group consisting of (i) a halogen, (ii) a C₁₋₆ alkyl group optionally substituted by 1 to 5 halogens, (iii) a C₆₋₁₄ aryl group, (iv) a hydroxyl group, (v) a C₁₋₆ alkoxy group optionally substituted by 1 to 5 halogens, (vi) a C₇₋₁₂ aralkyloxy group and (vii) a C₁₋₅ alkoxy-carbonyl group, or a salt thereof.

6. The compound of claim 4, wherein R is (1) a C₁₋₆ alkyl group optionally substituted by a C₆₋₁₄ aryl group or (2) a C₆₋₁₄ aryl group, or a salt thereof.

7. The compound of claim 4, wherein R is a phenyl group, or a salt thereof.

8. The compound of claim 4, wherein R is a methyl group or a tert-butyl group, or a salt thereof.

9. The compound of claim 1, which is an (R)-form represented by the formula 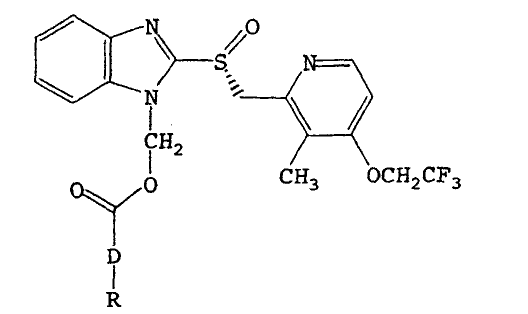 wherein each symbol is as defined in claim 1, or a salt thereof.

10. A production method for the compound of claim 1 or a salt thereof, which comprises (1) condensing a compound represented by the formula (II) 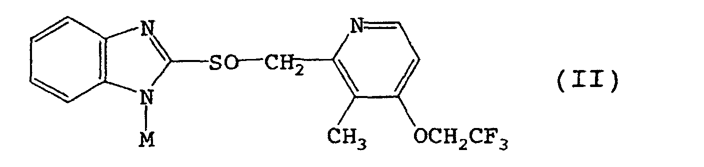 wherein M is a hydrogen atom, a metal cation or a quaternary ammonium ion, or a salt thereof, with a compound represented by the formula (III): R-D-C(=O)-O-CH₂-X wherein X is a halogen, D is an oxygen atom or a bond, and R is a hydrocarbon group optionally having substituents, or (2) condensing a compound represented by the formula (IV) 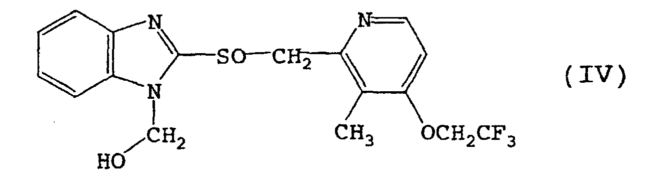 with carboxylic acid represented by the formula: R-D-COOH wherein each symbol is as defined above or a reactive derivative thereof, or
(3) subjecting a compound represented by the formula (V) 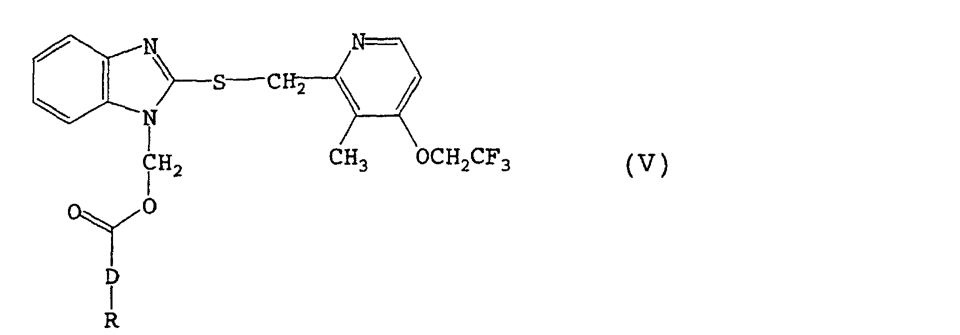 wherein each symbol is as defined above, or a salt thereof, to oxidation reaction.

11. A pharmaceutical composition comprising the compound of claim 1 or 4.

12. The pharmaceutical composition of claim 11, which is an agent for the prophylaxis or treatment of digestive ulcer, gastritis, reflux esophagitis, NUD, gastric cancer, gastric MALT lymphoma, gastric hyperacidity or upper gastrointestinal hemorrhage.

13. A commercial package comprising a pharmaceutical composition of claim 12 and written matter associated therewith, the written matter stating that the pharmaceutical composition can or should be used for the prophylaxis or treatment of digestive ulcer, gastritis, reflux esophagitis, NUD, gastric cancer, gastric MALT lymphoma, gastric hyperacidity or upper gastrointestinal hemorrhage.

14. The pharmaceutical composition of claim 11, which is an agent for the eradication of *Helicobacter pylori.*

15. A commercial package comprising a pharmaceutical composition of claim 14 and written matter associated therewith, the written matter stating that the pharmaceutical composition can or should be used for eradicating *Helicobacter pylori.*

16. An agent for the prophylaxis or treatment of digestive ulcer, gastritis, reflux esophagitis, NUD, gastric cancer, gastric MALT lymphoma, gastric hyperacidity or upper gastrointestinal hemorrhage, which comprises the compound of claim 1 as an active ingredient.

17. An agent for the eradication of *Helicobacter pylori,* which comprises the compound of claim 1 as an active ingredient.

18. A method for the prophylaxis or treatment of digestive ulcer, gastritis, reflux esophagitis, NUD, gastric cancer, gastric MALT lymphoma, gastric hyperacidity or upper gastrointestinal hemorrhage, which comprises administering the compound of claim 1.

19. A method for eradicating *Helicobacter pylori,* which comprises administering the compound of claim 1.

20. Use of the compound of claim 1 for the production of an agent for the prophylaxis or therapy of digestive ulcer, gastritis, reflux esophagitis, NUD, gastric cancer, gastric MALT lymphoma, gastric hyperacidity or upper gastrointestinal hemorrhage.

21. Use of the compound of claim 1 for the production of an agent for the eradication of *Helicobacter pylori.*
